# EUROPEAN PATENT APPLICATION

(11) **EP 4 512 803 A1**
(43) Date of publication of application: **26.02.2025**
(21) Application number: 23791233.2
(22) Date of filing: 18.04.2023
(51) Int. Cl.: C07D 307/79, C07D 333/62, C07D 209/10, A61K 31/343, A61K 31/404, A61K 31/381, A61P 3/00, A61P 35/00, A61P 37/02

(54) **GPCR REGULATOR AND USE THEREOF**

(30) Priority: 18.04.2022 CN 202210404732
(71) Applicant: Beijing Changping Laboratory, Changping District Beijing 102206 (CN); Peking University, Beijing 100871 (CN)
(72) Inventor: JIAO, Ning, Beijing 102206 (CN); SUN, Jinpeng, Beijing 100871 (CN); YU, Xiao, Beijing 100871 (CN); DOU, Xiaodong, Beijing 102206 (CN); XU, Guofeng, Beijing 102206 (CN); CHENG, Jie, Beijing 100871 (CN); HUO, Tongyu, Beijing 100871 (CN); GAO, Mingxin, Beijing 100871 (CN); ZHAO, Xinyi, Beijing 100871 (CN); WANG, Jiale, Beijing 100871 (CN); ZHANG, Caifang, Beijing 100871 (CN); LIU, Yameng, Beijing 102206 (CN)
(74) Representative: Manfredi, Irene
(86) International application number: PCT/CN2023/088997
(87) International publication number: WO 2023/202578

(57) **Abstract**

The present invention relates to a GPCR modulator and use thereof, and particularly provides a compound represented by Formula (I), or a stereoisomer, a prodrug, a crystal form, a pharmaceutically acceptable salt, a pharmaceutically acceptable solvate or a pharmaceutically acceptable ester of the compound.

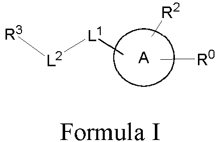

## Description

### Technical Field

The present invention relates to the field of medicine, and specifically to a GPCR modulator and use thereof.

### Background Art

G protein-coupled receptors (GPCRs) are a group of transmembrane proteins that transmit chemical signals from extracellular matrix proteins into cells, and participate in and regulate the operation of physiological and pathological functions. More than 800 GPCRs have been identified in the human genome, which are divided into A, B, C, and F classes based on the similarity of amino acid sequences. Among them, the class A (also known as rhodopsin-like GPCR) has the largest family members, consisting of 719 GPCRs. GPCR signal regulation involves ligands, GPCRs, effector proteins, and downstream corresponding signal pathways. After the ligands acts on GPCRs, GPCRs are activated and undergo conformational rearrangement, recruiting effector proteins to exert signal transduction functions. GPCR signal transduction has a high degree of biological complexity and importance, so GPCRs have become very valuable drug targets.

G protein-coupled receptor 132 (GPR132) is a seven-transmembrane G protein-coupled receptor, and a GPCR of the rhodopsin family (Class A). In 1998, Weng et al. found that B cells and T cells can upregulate GPR132 expression under stress induction, causing G2 phase cell cycle arrest. Therefore, the gene encoding this protein is named G2A (G2 accumulation) and is considered to be a potential tumor suppressor gene. GPR132 expression is tissue-specific and cell-type-specific, mainly present in blood and lymphoid tissues. GPR132 expression is mainly found in blood cells such as macrophages, dendritic cells, neutrophils, T cells, B lymphocytes, but its expression in immune cells is not specific.

As far as people know, GPR132 mainly plays a role in immune cells, regulates immune function, and is closely related to a variety of immune-related diseases and malignant tumors. In 2001, Le et al. found that GPR132-deficient mice developed delayed autoimmune syndrome and proposed that GPR132 is a potential target for autoimmune diseases. David et al. found that GPR132 deficiency promoted macrophage activation and aggravated atherosclerotic diseases. In the inflammatory bowel disease model induced by sodium dextran sulfate, Frasch et al. found that IFN-γ expression decreased after monocytes lost GPR132, aggravating the progression of colitis. In addition to immune diseases, GPR132 may play an important role in the hematopoietic system. Li P et al. found that the GPR132 agonist 11,12-EET significantly increased the mRNA expression of hematopoietic genes in the zebrafish model; in the mouse bone marrow transplantation model, the use of 11,12-EET to treat bone marrow cells significantly improved the short-term and long-term chimerism of the transplanted donor. Lahvic JL et al. used the GPR132 knockout mouse model to find that GPR132 in bone marrow transplantation improved the chimerism of donor cells by promoting the implantation and homing of donor hematopoietic stem cells and progenitor cells. Therefore, GPR132 agonists are expected to be used in autoimmune diseases and hematopoietic stem cell transplantation. In addition, Takenobu et al. found that the use of small molecule ONC212 to stimulate GPR132 could promote apoptosis of acute myeloid leukemia cells and prolong the survival of mice with transplanted tumors.

However, those skilled in the art are still eager to obtain a new type of GPR132 agonist useful in diseases such as atherosclerosis, colorectal inflammation and leukemia, or in treatment approaches such as bone marrow transplantation.

### Description of the Invention

The inventors of the present application have obtained a GPR132 modulator (e.g., agonist) with new structure through in-depth research and creative discovery, which can be used in the prevention and/or treatment of diseases such as atherosclerosis, colorectal inflammation, leukemia, or in treatment approaches such as bone marrow transplantation.

To this end, in the first aspect of the present invention, the present invention provides a compound of Formula I, or a stereoisomer, prodrug, crystal form, pharmaceutically acceptable salt, pharmaceutically acceptable solvate or pharmaceutically acceptable ester of the compound, 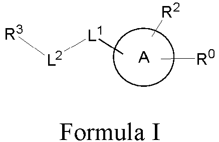 wherein:
Ring A is selected from the group consisting of benzofuran ring, benzothiophene ring, indole ring and benzene ring;
R⁰ is selected from the group consisting of 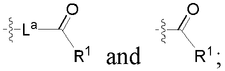
L^{a} is selected from the group consisting of C1-C6 alkylene;
R¹ is selected from the group consisting of hydroxyl, C1-C6 alkoxy and -NH-OH;
R² is selected from the group consisting of hydrogen, halogen and C1-C6 alkyl;
R³ is selected from the group consisting of C2-C6 alkenyl, C2-C6 alkynyl, phenyl, naphthyl and 5- to 10-membered heteroaryl; optionally, the phenyl, naphthyl and 5- to 10-membered heteroaryl are each independently substituted by 1 to 5 groups selected from the group consisting of R^{x}, or the phenyl, naphthyl and 5- to 10-membered heteroaryl are each independently substituted by 2 adjacent groups, and the 2 adjacent groups and the carbon atoms to which they are respectively connected together form a 5- to 6-membered heterocyclic ring;
R^{x} is selected from the group consisting of C1-C6 alkyl, halogen, C1-C6 alkoxy, C1-C6 haloalkyl, C2-C6 alkynyl, cyano, phenyl and -NR^{m}Rⁿ;
R^{m} and Rⁿ are each independently selected from the group consisting of hydrogen and C1-C6 alkyl;
L¹ is selected from the group consisting of 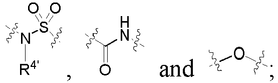
preferably, L¹ is selected from the group consisting of 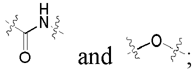
L² is selected from the group consisting of 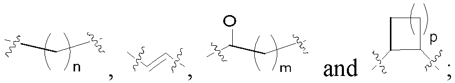
n is selected from the group consisting of 1, 2, 3, 4, 5 and 6;
m is selected from the group consisting of 1, 2, 3, 4, 5 and 6;
p is selected from the group consisting of 0, 1, 2 and 3;
R^{4'} is selected from the group consisting of phenyl-(CH₂)ᵣ- and 5- to 10-membered heteroaryl-(CH₂)ᵣ-, optionally, the phenyl and 5- to 10-membered heteroaryl are each independently substituted by 1 to 5 groups selected from the group consisting of R^{a};
r is selected from the group consisting of 0, 1, 2, 3, 4 and 5;
R^{a} is selected from the group consisting of halogen, C1-C6 alkyl-O-C(=O)-, and 5- to 6-membered saturated heterocyclyl optionally substituted by 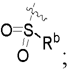
R^{b} is selected from the group consisting of hydrogen and C1-C6 alkyl.

In some embodiments, Ring A is selected from the group consisting of 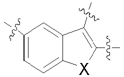 and 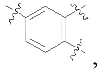 X is selected from the group consisting of O, S and NH, and X is preferably O.

In some embodiments, R⁰ is

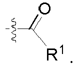

**In** some embodiments, L^{a} is selected from the group consisting of 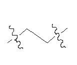 and 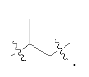 .

In some embodiments, R¹ is selected from the group consisting of hydroxyl and C1-C6 alkoxy.

In some embodiments, R¹ is selected from the group consisting of hydroxyl and ethoxy.

In some embodiments, R¹ is hydroxyl.

In some embodiments, R¹ is selected from the group consisting of hydroxyl, ethoxy and - NH-OH.

In some embodiments, R² is selected from the group consisting of halogen and C1-C6 alkyl.

In some embodiments, R² is selected from the group consisting of fluorine, chlorine and methyl.

In some embodiments, R² is selected from the group consisting of C1-C6 alkyl.

In some embodiments, R² is methyl.

In some embodiments, R³ is selected from the group consisting of C2-C6 alkenyl, C2-C6 alkynyl, phenyl, naphthyl, pyridyl, pyrrolyl, thienyl and indolyl; optionally, the phenyl, naphthyl, pyridyl, pyrrolyl, thienyl and indolyl are each independently substituted by 1 to 2 (preferably 1) groups selected from the group consisting of R^{x}, or the phenyl, naphthyl, pyridyl, pyrrolyl, thienyl and indolyl are each independently substituted by 2 adjacent groups, and the 2 adjacent groups and the carbon atoms to which they are respectively connected together form a 5- to 6-membered heterocyclic ring.

In some embodiments, R³ is selected from the group consisting of C2-C6 alkenyl, C2-C6 alkynyl, phenyl, naphthyl, pyridyl, pyrrolyl, thienyl and indolyl; optionally, the phenyl, pyridyl and thienyl are each independently substituted by 1 to 2 (preferably 1) groups selected from the group consisting of R^{x}, or the phenyl is substituted by 2 adjacent groups, and the 2 adjacent groups and the carbon atoms to which they are respectively connected form

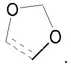

In some embodiments, R³ is selected from the group consisting of:
1) 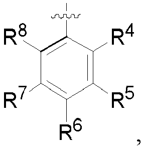 wherein:
   R⁴, R⁵, R⁶, R⁷ and R⁸ are each independently selected from the group consisting of hydrogen, C1-C6 alkyl, halogen, C1-C6 alkoxy, C1-C6 haloalkyl, C2-C6 alkynyl, cyano, phenyl and -NR^{m}Rⁿ; or, R⁴, R⁵ and the carbon atoms to which they are respectively connected together form a 5- to 6-membered heterocyclic ring, and R⁶, R⁷, R⁸ are hydrogen;
   preferably, any one of R⁴, R⁵, R⁶, R⁷, and R⁸ (e.g., R⁵, R⁶ or R⁷) is selected from the group consisting of hydrogen, C1-C6 alkyl, halogen, C1-C6 alkoxy, C1-C6 haloalkyl, C2-C6 alkynyl, cyano, phenyl and -NR^{m}Rⁿ, and the rest are hydrogen;
   more preferably, any one of R⁴, R⁵, R⁶, R⁷, and R⁸ (e.g., R⁵, R⁶ or R⁷) is selected from the group consisting of hydrogen, methyl, fluorine, chlorine, iodine, methoxy, 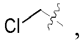 trifluoromethyl, 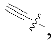 cyano, phenyl and 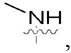 and the rest are hydrogen;
   or preferably, R⁴, R⁵ and the carbon atoms to which they are respectively connected together form 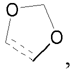 and R⁶, R⁷ and R⁸ are hydrogen;
2) 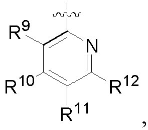 wherein:
   R⁹, R¹⁰, R¹¹ and R¹² are each independently selected from the group consisting of hydrogen, C1-C6 alkyl, halogen and C1-C6 alkoxy;
   preferably, R⁹, R¹⁰, R¹¹ and R¹² are each independently selected from the group consisting of hydrogen, halogen and C1-C6 alkoxy;
   more preferably, any one of R⁹, R¹⁰, R¹¹ and R¹² (e.g., R¹⁰ or R¹¹) is selected from the group consisting of hydrogen, halogen and C1-C6 alkoxy, and the rest are hydrogen;
   most preferably, any one of R⁹, R¹⁰, R¹¹ and R¹² (e.g., R¹⁰ or R¹¹) is selected from the group consisting of hydrogen, chlorine and methoxy, and the rest are hydrogen;
3) 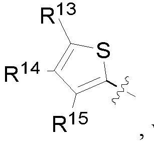 wherein:
   R¹³, R¹⁴ and R¹⁵ are each independently selected from the group consisting of hydrogen, C1-C6 alkyl and halogen;
   preferably, any one of R¹³, R¹⁴ and R¹⁵ (e.g., R¹³) is selected from the group consisting of hydrogen, C1-C6 alkyl and halogen, and the rest are hydrogen;
   more preferably, any one of R¹³, R¹⁴ and R¹⁵ (e.g., R¹³) is selected from the group consisting of hydrogen, methyl and chlorine, and the rest are hydrogen;
4)

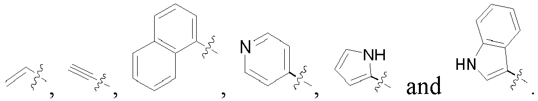

In some embodiments, R³ is selected from the group consisting of

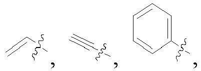

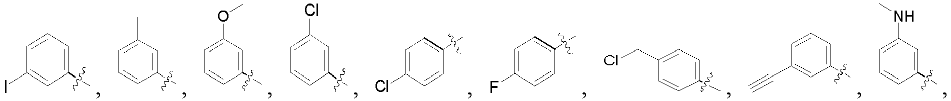

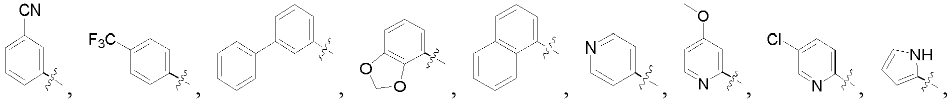

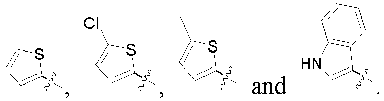

In some embodiments, R^{x} is selected from the group consisting of methyl, fluorine, chlorine, iodine, methoxy, 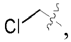 trifluoromethyl, 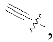 cyano, phenyl and -NR^{m}Rⁿ.

In some embodiments, among R^{m} and Rⁿ, any one is hydrogen, and the other is selected from the group consisting of C1-C6 alkyl.

In some embodiments, among R^{m} and Rⁿ, any one is hydrogen, and the other is methyl.

In some embodiments, R^{4'} is selected from the group consisting of phenyl-(CH₂)ᵣ- and quinolyl-(CH₂)ᵣ-, and optionally, the phenyl and quinolyl are each independently substituted by a group selected from the group consisting of R^{a}.

In some embodiments, R^{4'} is selected from the group consisting of phenyl-(CH₂)ᵣ- and quinolyl-(CH₂)ᵣ-, and optionally, the phenyl is substituted by a group selected from the group consisting of R^{a}.

In some embodiments, r is selected from the group consisting of 0 and 1.

In some embodiments, R^{a} is selected from the group consisting of halogen, C1-C6 alkyl-O-C(=O)-, and piperazinyl which is substituted by

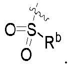

In some embodiments, R^{a} is selected from the group consisting of halogen, C1-C6 alkyl-O-C(=O)-, and piperazinyl which is substituted by

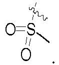

In some embodiments, R^{a} is selected from the group consisting of bromine, 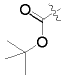 and

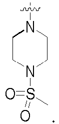

In some embodiments, R^{b} is selected from the group consisting of C1-C6 alkyl.

In some embodiments, R^{b} is methyl.

In some embodiments, R^{4'} is selected from the group consisting of 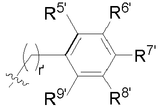 and 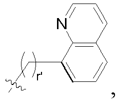 wherein,
r' is selected from the group consisting of 0, 1, 2, 3, 4 and 5;
preferably, r' is selected from the group consisting of 0 and 1;
R^{5'}, R^{6'}, R^{7'}, R^{8'} and R^{9'} are each independently selected from the group consisting of hydrogen, halogen, C1-C6 alkyl-O-C(=O)-, and 5- to 6-membered saturated heterocyclyl optionally substituted by 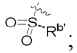
preferably, among R^{5'}, R^{6'}, R^{7'}, R^{8'} and R^{9'}, any one (e.g., R^{7'}) is selected from the group consisting of hydrogen, halogen, C1-C6 alkyl-O-C(=O)-, and 5- to 6-membered saturated heterocyclyl optionally substituted by 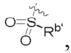 and the rest are hydrogen;
more preferably, among R^{5'}, R^{6'}, R^{7'}, R^{8'}, R^{9'} , any one (e.g., R^{7'}) is selected from the group consisting of hydrogen, halogen, C1-C6 alkyl-O-C(=O)-, and piperazinyl which is substituted by 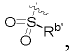 and the rest are hydrogen;
further preferably, among R^{5'}, R^{6'}, R^{7'}, R^{8'}, and R^{9'}, any one (e.g., R^{7'}) is selected from the group consisting of hydrogen, halogen, C1-C6 alkyl-O-C(=O)-, and piperazinyl which is substituted by 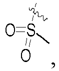 and the rest are hydrogen;
most preferably, among R^{5'}, R^{6'}, R^{7'}, R^{8'}, and R^{9'}, any one (e.g., R^{7'}) is selected from the group consisting of hydrogen, bromine, 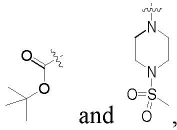 and the rest are hydrogen;
R^{b'} is selected from the group consisting of hydrogen and C1-C6 alkyl;
preferably, R^{b'} is selected from the group consisting of C1-C6 alkyl;
more preferably, R^{b'} is methyl.

In some embodiments, R^{4'} is selected from the group consisting of

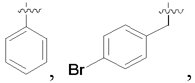

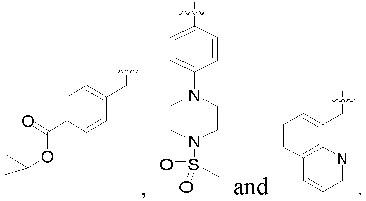

In some embodiments, L¹ is selected from the group consisting of 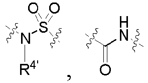 and 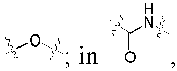 the carbonyl end is connected to L², and the amino end is connected to the benzene ring; and in 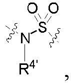 the amino end is connected to L², and the acyl end is connected to the benzene ring.

In some embodiments, L¹ is 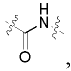 and in 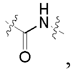 the carbonyl end is connected to L², and the amino end is connected to the benzene ring.

In some embodiments, L² is selected from the group consisting of 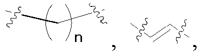 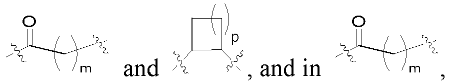 the carbonyl end is connected to R³.

In some embodiments, L² is selected from the group consisting of 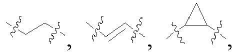 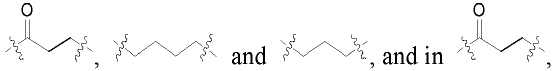 the carbonyl end is connected to R³.

In some embodiments, L² is selected from the group consisting of 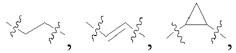 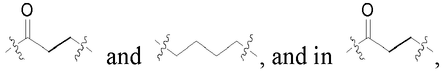 the carbonyl end is connected to R³.

In some embodiments, L² is

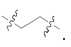

In some embodiments, n is selected from the group consisting of 2, 3 and 4.

In some embodiments, n is selected from the group consisting of 2 and 4.

In some embodiments, m is selected from the group consisting of 1, 2 and 3.

In some embodiments, m is 2.

In some embodiments, p is 0.

In the second aspect of the present invention, the present invention provides a compound represented by Formula I-1, Formula I-2 or Formula I-3, or a stereoisomer, prodrug, crystal form, pharmaceutically acceptable salt, pharmaceutically acceptable solvate or pharmaceutically acceptable ester of the compound, 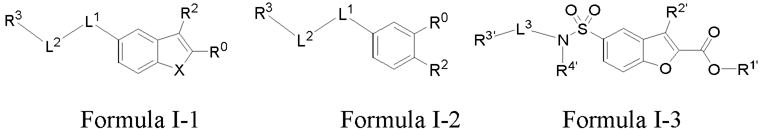 wherein:
X is selected from the group consisting of O, S and NH, preferably O;
R⁰, R², R³, L¹ and L² are defined as described in the first aspect;
R^{1'} is selected from the group consisting of hydrogen and C1-C6 alkyl;
R^{2'} is selected from the group consisting of hydrogen and C1-C6 alkyl;
R^{3'} is phenyl, optionally, the phenyl is substituted by 1 to 5 groups selected from the group consisting of C1-C6 alkyl;
L³ is selected from 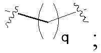
q is selected from the group consisting of 1, 2, 3, 4, 5 and 6;
R^{4'} is selected from the group consisting of phenyl-(CH₂)ᵣ- and 5- to 10-membered heteroaryl-(CH₂)ᵣ-, optionally, the phenyl and 5- to 10-membered heteroaryl are each independently substituted by 1 to 5 groups selected from the group consisting of R^{a};
r is selected from the group consisting of 0, 1, 2, 3, 4 and 5;
R^{a} is selected from the group consisting of halogen, C1-C6 alkyl-O-C(=O)-, and 5- to 6-membered saturated heterocyclyl optionally substituted by 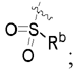
R^{b} is selected from the group consisting of hydrogen and C1-C6 alkyl.

In some embodiments, R^{1'} is selected from the group consisting of hydrogen and ethyl.

In some embodiments, R^{1'} is hydrogen.

In some embodiments, R^{2'} is selected from the group consisting of C1-C6 alkyl.

In some embodiments, R^{2'} is methyl.

In some embodiments, R^{3'} is phenyl, optionally, the phenyl is substituted by 1 group selected from the group consisting of C1-C6 alkyl.

In some embodiments, R^{3'} is phenyl.

In some embodiments, q is selected from the group consisting of 1, 2 and 3.

In some embodiments, q is 2.

In some embodiments, R^{4'} is selected from the group consisting of phenyl-(CH₂)ᵣ- and quinolyl-(CH₂)ᵣ-, optionally, the phenyl and quinolyl is each independently substituted by 1 group selected from the group consisting of R^{a}.

In some embodiments, R^{4'} is selected from the group consisting of phenyl-(CH₂)ᵣ- and quinolyl-(CH₂)ᵣ-, optionally, the phenyl group is substituted by 1 group selected from the group consisting of R^{a}.

In some embodiments, r is selected from the group consisting of 0 and 1.

In some embodiments, R^{a} is selected from the group consisting of halogen, C1-C6 alkyl-O-C(=O)-, and piperazinyl which is substituted by

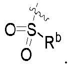

In some embodiments, R^{a} is selected from the group consisting of halogen, C1-C6 alkyl-O-C(=O)-, and piperazinyl which is substituted by

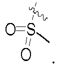

In some embodiments, R^{a} is selected from the group consisting of bromine, 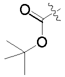 and

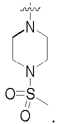

In some embodiments, R^{b} is selected from the group consisting of C1-C6 alkyl.

In some embodiments, R^{b} is methyl.

In some embodiments, R^{4'} is selected from the group consisting of 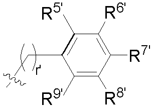 and 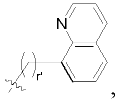 wherein,
r' is selected from the group consisting of 0, 1, 2, 3, 4 and 5;
preferably, r' is selected from the group consisting of 0 and 1;
R^{5'}, R^{6'}, R^{7'}, R^{8'} and R^{9'} are each independently selected from the group consisting of hydrogen, halogen, C1-C6 alkyl-O-C(=O)-, and 5- to 6-membered saturated heterocyclyl optionally substituted by 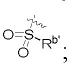
preferably, among R^{5'}, R^{6'}, R^{7'}, R⁸ and R^{9'}, any one (e.g., R^{7'}) is selected from the group consisting of hydrogen, halogen, C1-C6 alkyl-O-C(=O)-, and 5- to 6-membered saturated heterocyclyl optionally substituted by 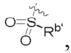 and the rest are hydrogen;
more preferably, among R^{5'}, R^{6'}, R^{7'}, R^{8'} and R^{9'} , any one (e.g., R^{7'}) is selected from the group consisting of hydrogen, halogen, C1-C6 alkyl-O-C(=O)-, and piperazinyl which is substituted by 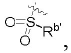 and the rest are hydrogen;
further preferably, among R^{5'}, R^{6'}, R^{7'}, R^{8'}, and R^{9'}, any one (e.g., R^{7'}) is selected from the group consisting of hydrogen, halogen, C1-C6 alkyl-O-C(=O)-, and piperazinyl which is substituted by 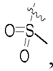 and the rest are hydrogen;
most preferably, among R^{5'}, R^{6'}, R^{7'}, R^{8'}, and R^{9'}, any one (e.g., R^{7'}) is selected from the group consisting of hydrogen, bromine, 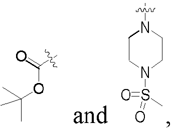 and the rest are hydrogen;
R^{b'} is selected from the group consisting of hydrogen and C1-C6 alkyl;
preferably, R^{b'} is selected from the group consisting of C1-C6 alkyl;
more preferably, R^{b'} is methyl.

In some embodiments, R^{4'} is selected from the group consisting of

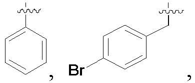

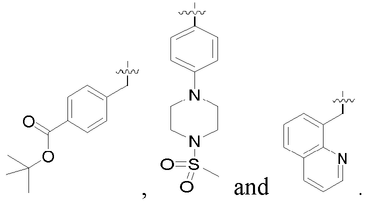

In the third aspect of the present invention, the present invention provides a compound represented by Formula I-1-1, or a stereoisomer, prodrug, crystal form, pharmaceutically acceptable salt, pharmaceutically acceptable solvate or pharmaceutically acceptable ester of the compound, 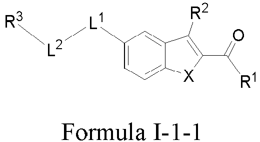 wherein:
X is selected from the group consisting of O, S and NH, and preferably O;
R¹, R², R³, L¹ and L² are defined as described in the first aspect.

In the fourth aspect of the present invention, the present invention provides a compound represented by Formula I-2-1, or a stereoisomer, prodrug, crystal form, pharmaceutically acceptable salt, pharmaceutically acceptable solvate or pharmaceutically acceptable ester of the compound, 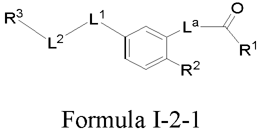 wherein:
R¹, R², R³, L^{a}, L¹ and L² are defined as described in the first aspect.

In some embodiments, R¹ is hydroxyl.

In some embodiments, R² is selected from the group consisting of halogen.

In some embodiments, R² is fluorine.

In some embodiments, R³ is

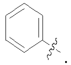

In some embodiments, L^{a} is selected from the group consisting of C1-C6 alkylene.

In some embodiments, L^{a} is selected from the group consisting of

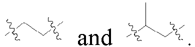

In some embodiments, L¹ is selected from the group consisting of 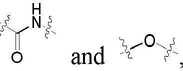 and in 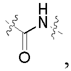 the carbonyl end is connected to L², and the amino end is connected to the benzene ring.

In some embodiments, L¹ is 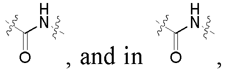 the carbonyl end is connected to L², and the amino end is connected to the benzene ring.

In some embodiments, L² is selected from

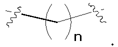

In some embodiments, L² is selected from the group consisting of

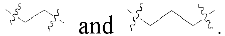

In some embodiments, n is selected from the group consisting of 1, 2, 3, 4, 5 and 6.

In some embodiments, n is selected from the group consisting of 2 and 3.

In some embodiments, the compound represented by Formula I is selected from the group consisting of the following:

| No. | Structural Formula | No. | Structural Formula |
|---|---|---|---|
| 1 | 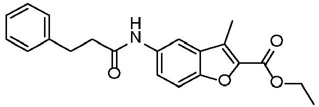 | 16 | 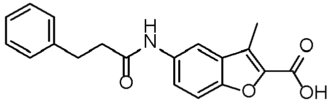 |
| 2 | 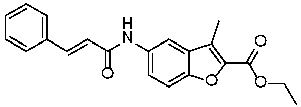 | 17 | 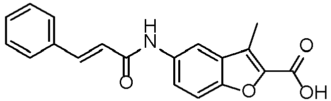 |
| 3 | 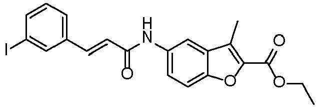 | 18 | 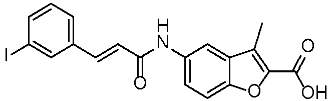 |
| 4 | 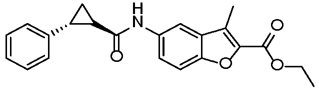 | 19 | 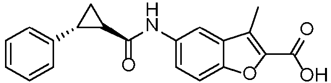 |
| 5 | 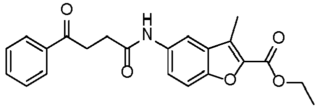 | 20 | 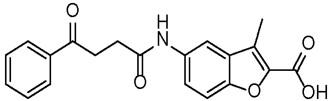 |
| 6 | 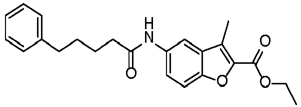 | 21 | 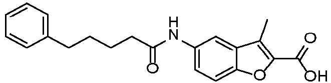 |
| 7 | 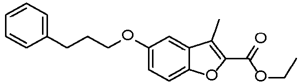 | 22 | 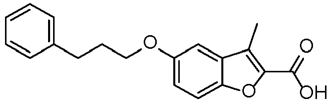 |
| B101 | 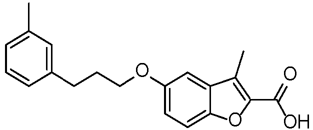 | B113 | 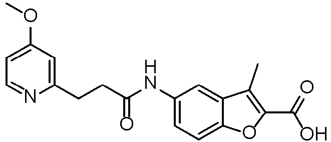 |
| B102 | 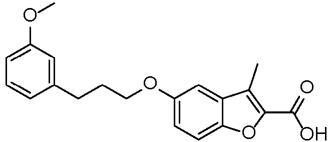 | B114 | 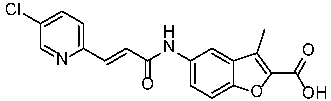 |
| B103 | 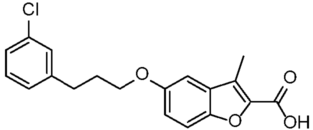 | B115 | 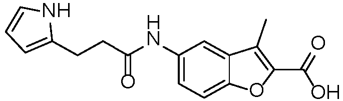 |
| GPR132-A-8 | 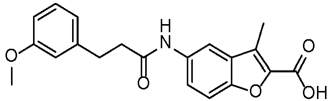 | B116 | 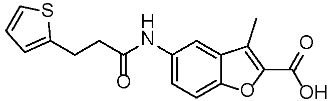 |
| B104 | 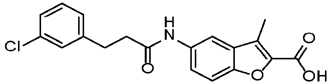 | B117 | 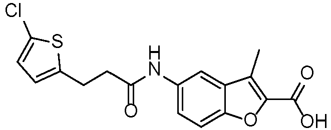 |
| B105 | 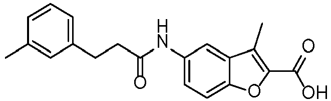 | B118 | 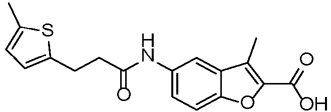 |
| B106 | 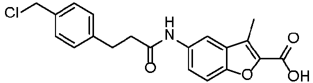 | B119 | 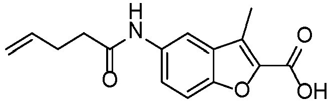 |
| B107 | 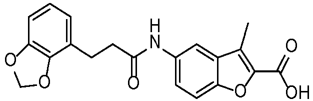 | B120 | 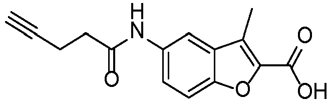 |
| B108 | 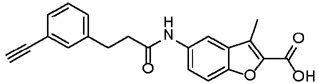 | B121 | 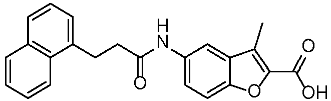 |
| B109 | 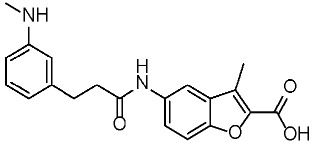 | B122 | 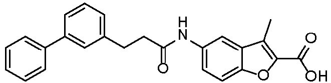 |
| B110 | 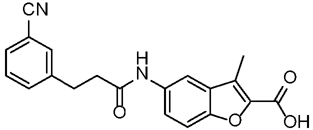 | B123 | 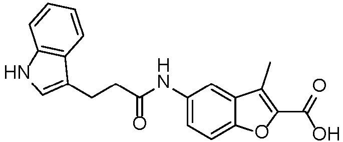 |
| B111 | 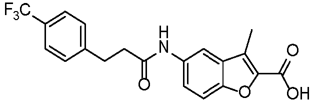 | B124 | 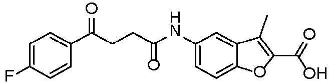 |
| B112 | 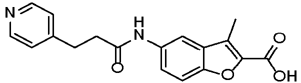 | B125 | 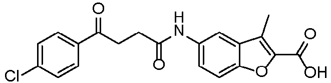 |
| B126 | 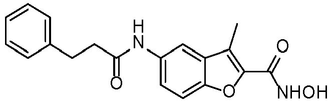 | B127 | 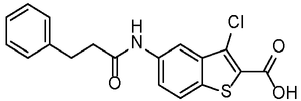 |
| B128 | 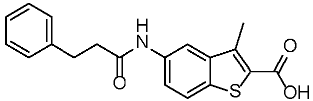 | B129 | 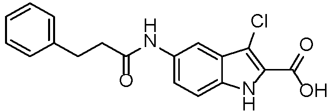 |
| B130 | 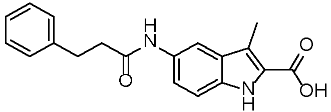 | B131 | 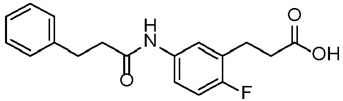 |
| B132 | 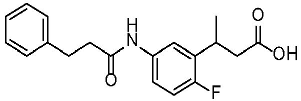 | B133 | 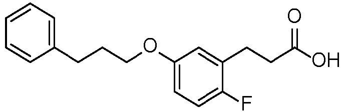 |
| 9 | 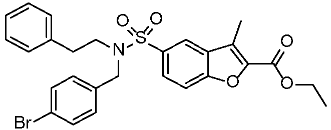 | 24 | 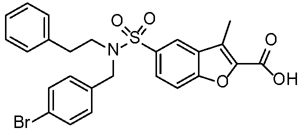 |
| 10 | 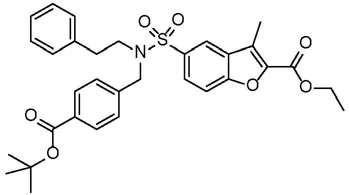 | 25 | 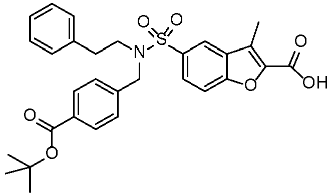 |
| 11 | 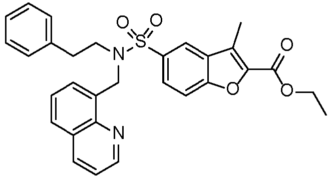 | 26 | 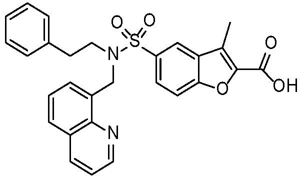 |
| 12 | 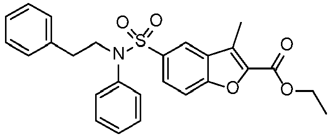 | 27 | 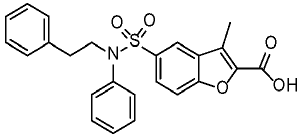 |
| 14 | 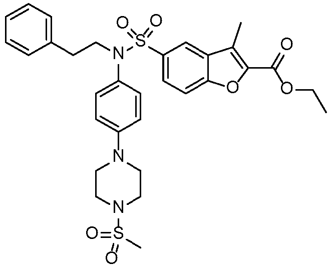 | 29 | 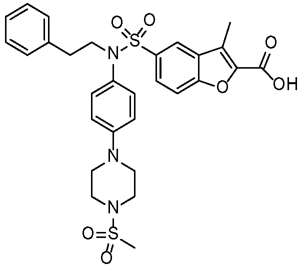 |

In the fifth aspect of the present invention, the present invention provides a pharmaceutical composition, which comprises the aforementioned compound, or a stereoisomer, prodrug, crystal form, pharmaceutically acceptable salt, pharmaceutically acceptable solvate or pharmaceutically acceptable ester of the compound, and an optional pharmaceutically acceptable excipient.

The compounds of the present invention can be used as GPR132 modulators (e.g., agonists), which can be used to prevent and/or treat diseases related to GPR132, or for hematopoietic stem cell transplantation, or for bone marrow transplantation, for example, used for the prevention and/or treatment of diseases such as atherosclerosis, colorectal inflammation, leukemia, or in treatment approaches such as bone marrow transplantation.

Thus, in the sixth aspect of the present invention, the present invention provides a use of the aforementioned compound or a stereoisomer, prodrug, crystal form, pharmaceutically acceptable salt, pharmaceutically acceptable solvate or pharmaceutically acceptable ester of the compound in manufacture of a medicament, or a use of the aforementioned pharmaceutical composition in manufacture of a medicament, or a use of the compound shown in Table A below or a stereoisomer, prodrug, crystal form, pharmaceutically acceptable salt, pharmaceutically acceptable solvate or pharmaceutically acceptable ester of the compound in manufacture of a medicament, in which the medicament is used for treatment and/or prevention of a disease related to GPR132, or for hematopoietic stem cell transplantation, or for bone marrow transplantation;

**Table A:**

| No. | Structural Formula | No. | Structural Formula |
|---|---|---|---|
| 8 | 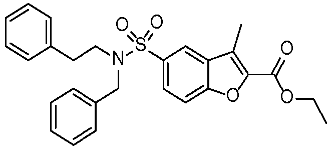 | 23 | 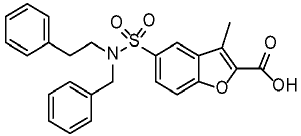 |
| 13 | 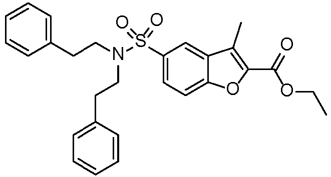 | 28 | 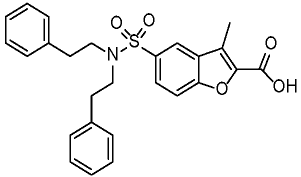 |
| 15 | 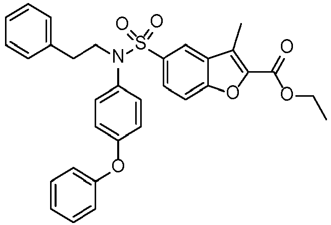 | 30 | 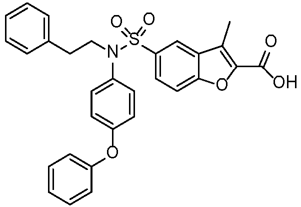 |

In some embodiments, the disease related to GPR132 is selected from the group consisting of tumor, metabolic disease, immune-related disease, and neuropathic pain.

In some embodiments, the tumor is selected from the group consisting of breast cancer, melanoma, meningioma, soft tissue sarcoma, salivary gland tumor, liver cancer (e.g., primary liver cancer), intraspinal tumor, mediastinal tumor, brain cancer, bone cancer, penile cancer, osteosarcoma, intracranial tumor, tongue cancer, maxillary sinus cancer, thyroid cancer, malignant lymphoma, multiple myeloma, pituitary adenoma, testicular tumor, non-Hodgkin's lymphoma, bladder cancer, leukemia (e.g., acute myeloid leukemia), gastric cancer, nasopharyngeal cancer, laryngeal cancer, oral cancer, esophageal cancer, lung cancer, kidney cancer, cervical cancer, choriocarcinoma, vulvar cancer, skin cancer, endometrial cancer, ovarian cancer, prostate cancer, pancreatic cancer, colon cancer, rectal cancer, colorectal cancer, Kaposi's sarcoma, non-melanoma skin cancer (including squamous cell carcinoma and basal cell carcinoma), hemangioma and glioma.

In some embodiments, the metabolic disease is selected from the group consisting of atherosclerosis, obesity, non-alcoholic fatty liver disease (NAFLD) (e.g., simple fatty liver or non-alcoholic steatohepatitis (NASH)), metabolic syndrome, type 2 diabetes, type 1 diabetes, insulin resistance, hyperinsulinemia, glucose intolerance, hyperglycemia, hyperlipidemia (e.g., hypercholesterolemia), and secondary complications of these diseases (e.g., diabetic complications such as retinopathy, neuropathy, nephropathy, and delayed wound healing, or cardiovascular and cerebrovascular diseases such as atherosclerosis, coronary heart disease, hypertension, and stroke).

In some embodiments, the immune-related disease is selected from the group consisting of:
secondary immunodeficiency: for example, secondary immunodeficiency caused by infection (e.g., rubella, measles, leprosy, tuberculosis, cytomegalovirus infection, HIV infection, coccidioidomycosis infection), protein loss (e.g., nephrotic syndrome, protein-losing enteropathy), insufficient immunoglobulin synthesis, lymphocyte loss (e.g., lymphocyte loss caused by drug and/or systemic infection), secondary immunodeficiency caused by other diseases (e.g., diabetes, cirrhosis, subacute sclerosing panencephalitis) and/or immunosuppressive therapy, and
autoimmune diseases: for example, inflammatory bowel disease, systemic lupus erythematosus, rheumatoid arthritis, scleroderma, hyperthyroidism, juvenile diabetes, idiopathic thrombocytic purpura, autoimmune hemolytic anemia, colorectal inflammation (e.g., ulcerative colitis), skin diseases, chronic liver diseases.

In the seventh aspect of the present invention, the present invention provides the aforementioned compound or a stereoisomer, prodrug, crystal form, pharmaceutically acceptable salt, pharmaceutically acceptable solvate or pharmaceutically acceptable ester of the compound, or the aforementioned pharmaceutical composition, or the compound shown in Table A above or a stereoisomer, prodrug, crystal form, pharmaceutically acceptable salt, pharmaceutically acceptable solvate or pharmaceutically acceptable ester of the compound, which is used for the treatment and/or prevention of a disease related to GPR132, or for hematopoietic stem cell transplantation, or for bone marrow transplantation.

In some embodiments, the disease related to GPR132 is selected from the group consisting of tumor, metabolic disease, immune-related disease, and neuropathic pain.

In some embodiments, the tumor is selected from the group consisting of breast cancer, melanoma, meningioma, soft tissue sarcoma, salivary gland tumor, liver cancer (e.g., primary liver cancer), intraspinal tumor, mediastinal tumor, brain cancer, bone cancer, penile cancer, osteosarcoma, intracranial tumor, tongue cancer, maxillary sinus cancer, thyroid cancer, malignant lymphoma, multiple myeloma, pituitary adenoma, testicular tumor, non-Hodgkin's lymphoma, bladder cancer, leukemia (e.g., acute myeloid leukemia), gastric cancer, nasopharyngeal cancer, laryngeal cancer, oral cancer, esophageal cancer, lung cancer, kidney cancer, cervical cancer, choriocarcinoma, vulvar cancer, skin cancer, endometrial cancer, ovarian cancer, prostate cancer, pancreatic cancer, colon cancer, rectal cancer, colorectal cancer, Kaposi's sarcoma, non-melanoma skin cancer (including squamous cell carcinoma and basal cell carcinoma), hemangioma and glioma.

In some embodiments, the metabolic disease is selected from the group consisting of atherosclerosis, obesity, non-alcoholic fatty liver disease (NAFLD) (e.g., simple fatty liver or non-alcoholic steatohepatitis (NASH)), metabolic syndrome, type 2 diabetes, type 1 diabetes, insulin resistance, hyperinsulinemia, glucose intolerance, hyperglycemia, hyperlipidemia (e.g., hypercholesterolemia), and secondary complications of these diseases (e.g., diabetic complications such as retinopathy, neuropathy, nephropathy, and delayed wound healing, or cardiovascular and cerebrovascular diseases such as atherosclerosis, coronary heart disease, hypertension, and stroke).

In some embodiments, the immune-related disease is selected from the group consisting of:
secondary immunodeficiency: for example, secondary immunodeficiency caused by infection (e.g., rubella, measles, leprosy, tuberculosis, cytomegalovirus infection, HIV infection, coccidioidomycosis infection), protein loss (e.g., nephrotic syndrome, protein-losing enteropathy), insufficient immunoglobulin synthesis, lymphocyte loss (e.g., lymphocyte loss caused by drug and/or systemic infection), secondary immunodeficiency caused by other diseases (e.g., diabetes, cirrhosis, subacute sclerosing panencephalitis) and/or immunosuppressive therapy, and
autoimmune diseases: for example, inflammatory bowel disease, systemic lupus erythematosus, rheumatoid arthritis, scleroderma, hyperthyroidism, juvenile diabetes, idiopathic thrombocytic purpura, autoimmune hemolytic anemia, colorectal inflammation (e.g., ulcerative colitis), skin diseases, chronic liver diseases.

In the eighth aspect of the present invention, the present invention provides a method for treating and/or preventing a disease related to GPR132, a method for performing hematopoietic stem cell transplantation, and a method for performing bone marrow transplantation, each of these methods independently comprises: administering to a subject an effective amount of the aforementioned compound or a stereoisomer, prodrug, crystal form, pharmaceutically acceptable salt, pharmaceutically acceptable solvate or pharmaceutically acceptable ester of the compound, or the aforementioned pharmaceutical composition, or a compound shown in Table A above or a stereoisomer, prodrug, crystal form, pharmaceutically acceptable salt, pharmaceutically acceptable solvate or pharmaceutically acceptable ester of the compound.

In some embodiments, the disease related to GPR132 is selected from the group consisting of tumor, metabolic disease, immune-related disease, and neuropathic pain.

In some embodiments, the tumor is selected from the group consisting of breast cancer, melanoma, meningioma, soft tissue sarcoma, salivary gland tumor, liver cancer (e.g., primary liver cancer), intraspinal tumor, mediastinal tumor, brain cancer, bone cancer, penile cancer, osteosarcoma, intracranial tumor, tongue cancer, maxillary sinus cancer, thyroid cancer, malignant lymphoma, multiple myeloma, pituitary adenoma, testicular tumor, non-Hodgkin's lymphoma, bladder cancer, leukemia (e.g., acute myeloid leukemia), gastric cancer, nasopharyngeal cancer, laryngeal cancer, oral cancer, esophageal cancer, lung cancer, kidney cancer, cervical cancer, choriocarcinoma, vulvar cancer, skin cancer, endometrial cancer, ovarian cancer, prostate cancer, pancreatic cancer, colon cancer, rectal cancer, colorectal cancer, Kaposi's sarcoma, non-melanoma skin cancer (including squamous cell carcinoma and basal cell carcinoma), hemangioma and glioma.

In some embodiments, the metabolic disease is selected from the group consisting of atherosclerosis, obesity, non-alcoholic fatty liver disease (NAFLD) (e.g., simple fatty liver or non-alcoholic steatohepatitis (NASH)), metabolic syndrome, type 2 diabetes, type 1 diabetes, insulin resistance, hyperinsulinemia, glucose intolerance, hyperglycemia, hyperlipidemia (e.g., hypercholesterolemia), and secondary complications of these diseases (e.g., diabetic complications such as retinopathy, neuropathy, nephropathy, and delayed wound healing, or cardiovascular and cerebrovascular diseases such as atherosclerosis, coronary heart disease, hypertension, and stroke).

In some embodiments, the immune-related disease is selected from the group consisting of:
secondary immunodeficiency: for example, secondary immunodeficiency caused by infection (e.g., rubella, measles, leprosy, tuberculosis, cytomegalovirus infection, HIV infection, coccidioidomycosis infection), protein loss (e.g., nephrotic syndrome, protein-losing enteropathy), insufficient immunoglobulin synthesis, lymphocyte loss (e.g., lymphocyte loss caused by drug and/or systemic infection), secondary immunodeficiency caused by other diseases (e.g., diabetes, cirrhosis, subacute sclerosing panencephalitis) and/or immunosuppressive therapy, and
autoimmune diseases: for example, inflammatory bowel disease, systemic lupus erythematosus, rheumatoid arthritis, scleroderma, hyperthyroidism, juvenile diabetes, idiopathic thrombocytic purpura, autoimmune hemolytic anemia, colorectal inflammation (e.g., ulcerative colitis), skin diseases, chronic liver diseases.

### Specific Models for Carrying Out the Invention

It should be understood that the terminology used herein is intended to describe specific embodiments and is not intended to be limiting. In addition, although any methods, devices and materials similar or equivalent to those described herein can be used to implement or test the present invention, preferred methods, devices and materials are now described.

In the present invention, unless otherwise explicitly stated, the expression "... each independently selected from" used herein can mean that the specific options expressed by the same or different symbols in different groups do not affect each other, or that the specific options expressed by the same or different symbols in the same group do not affect each other.

The substituents of the compounds of the present invention are disclosed according to the group types or ranges. In particular, the present invention comprises each independent subcombination of the individual members of these group types and ranges. For example, the term "C1-C6 alkyl" specifically refers to the independently disclosed methyl, ethyl, C3 alkyl, C4 alkyl, C5 alkyl and C6 alkyl.

The term "alkyl" refers to branched and straight-chain saturated aliphatic hydrocarbonyl groups including those with a specified number of carbon atoms. For example, "C1-C6 alkyl" refers to C1, C2, C3, C4, C5 and C6. In addition, for example, "C1-C6 alkyl" refers to alkyl groups having 1 to 6 carbon atoms, preferably "C1-C4 alkyl", and more preferably "C1-C3 alkyl". Examples of alkyl groups include, but are not limited to, methyl, ethyl, propyl (e.g., *N-*propyl, isopropyl), butyl (e.g., *N-*butyl, isobutyl, tert-butyl), pentyl (e.g., *N*-pentyl, isopentyl, neopentyl), etc.

The term "alkylene" refers to a divalent group obtained by removing one hydrogen atom from any of the above alkyl groups (e.g., C1-C6 alkyl, C1-C4 alkyl, C1-C3 alkyl, etc.), such as

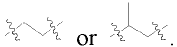

The term "alkoxy" refers to any of the above alkyl groups (e.g., C1-C6 alkyl, C1-C4 alkyl, C1-C3 alkyl, etc.) connected via an oxygen atom (-O-) to the rest of the molecule.

The term "C1-C6 haloalkyl" refers to a group obtained by replacing one or more hydrogen atoms in any of the above alkyl groups (e.g., C1-C6 alkyl, C1-C4 alkyl, C1-C3 alkyl, etc.) with a halogen (preferably fluorine or chlorine), such as monofluoromethyl, difluoroethyl, trifluoromethyl, 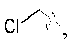 etc.

The term "C2-C6 alkenyl" refers to any straight or branched group containing 2-6 carbon atoms and at least one carbon-carbon double bond, such as vinyl, 1-propenyl, 2-propenyl, etc.

The term "C2-C6 alkynyl" refers to any straight or branched group containing 2-6 carbon atoms and at least one carbon-carbon triple bond, such as ethynyl, 1-propynyl, 2-propynyl, etc.

Heteroatom refers to N, O or S.

Halogen refers to fluorine, chlorine, bromine or iodine.

The term "heteroaryl" refers to a substituted and unsubstituted aromatic 5-membered or 6-membered monocyclic group, 8-membered, 9-membered or 10-membered bicyclic group and 11-membered to 14-membered tricyclic group having at least one heteroatom (N, O or S) in at least one ring, and the heteroatom-containing ring optionally further has 1, 2 or 3 heteroatoms selected from the group consisting of N, O and S. Among them, substituted and unsubstituted aromatic 8-membered, 9-membered or 10-membered bicyclic group and 11-membered to 14-membered tricyclic group having at least one heteroatom (N, O or S) in at least one ring are "fused heteroaryl". For a bicyclic or tricyclic heteroaryl group, its bicyclic or tricyclic overall structure is required to form an aromatic system. The heteroaryl group can be connected to any available nitrogen or carbon atom of any ring. And those skilled in the art can understand that two adjacent atoms (preferably carbon atoms) are shared between each two rings in the fused ring.

Specifically, the "5- to 10-membered heteroaryl" can be a 5-membered or 6-membered monocyclic heteroaryl group, or an 8-membered, 9-membered or 10-membered bicyclic heteroaryl group.

Exemplary monocyclic heteroaryl groups include, but are not limited to: pyrrolyl, pyrazolyl, imidazolyl, oxazolyl, isoxazolyl, thiazolyl, thiadiazolyl, isothiazolyl, furanyl, thienyl, oxadiazolyl, pyridyl, pyrazinyl, pyrimidinyl, pyridazinyl, triazinyl, triazolyl, etc.

Exemplary bicyclic heteroaryl groups include, but are not limited to, indolyl, 5-azaindolyl, pyrrolo[2,3-d]pyrimidinyl, 5,6-diazaindolyl, 6-azaindolyl, 7-azaindolyl, pyrazolo[3,4-b]pyridinyl, pyrrolo[2,3-c]pyridazinyl, thieno[2,3-d]imidazolyl, thieno[2,3-d]imidazolyl, pyrazolo[3,4-c]pyridinyl, benzothiazolyl, benzimidazolyl, benzoxazolyl, benzothienyl, quinolyl, isoquinolyl, benzofuranyl, indolizinyl, quinoxalinyl, indazolyl, pyrrolopyrimidinyl, furopyridinyl, isoindolyl, and the like.

The terms "heterocycle", "heterocyclic" or "heterocyclyl" are used interchangeably and refer to substituted and unsubstituted 3- to 7-membered (preferably 4- to 7-membered, more preferably 5- to 6-membered) monocyclic groups, 7- to 11-membered bicyclic groups and 10-to 15-membered tricyclic groups, which may contain one or more double bonds but do not constitute aromatic ring; and wherein at least one ring has at least one heteroatom (N, O or S). The fused rings completing the bicyclic and tricyclic groups may contain only carbon atoms and may be saturated or partially saturated and do not constitute aromatic rings. The heterocyclyl may be attached at any available nitrogen or carbon atom.

Exemplary monocyclic heterocyclyls include azacyclobutyl, oxacyclobutyl, pyrrolidinyl, imidazolinyl, oxazolidinyl, isoxazolinyl, thiazolidinyl, tetrahydrofuranyl, piperidinyl, piperazinyl, 2-oxopiperazinyl, 2-oxopiperidinyl, 2-oxopyrrolidinyl, 2-oxazacycloheptatrienyl, 1-pyridonyl, 4-piperidonyl, tetrahydropyranyl, morpholinyl, 1,3-dioxolane, etc.

Wherein, "saturated heterocyclyl" means that the heterocyclic ring defined above does not contain unsaturated bond, such as double bond. For example, "5- to 6-membered saturated heterocyclyl" can be pyrrolidinyl, oxazolidinyl, thiazolidinyl, tetrahydrofuranyl, piperidinyl, piperazinyl, tetrahydropyranyl, morpholinyl, 1,3-dioxolane, preferably piperidinyl and piperazinyl, more preferably piperazinyl.

The term "substituted" means that any one or more hydrogen atoms on a designated atom or group are replaced by a selected designated group, provided that the normal valence of the designated atom is not exceeded.

In the present invention, the expression "phenyl-(CH₂)ᵣ- and 5- to 10-membered heteroaryl-(CH₂)ᵣ-, optionally, the phenyl and 5- to 10-membered heteroaryl are each independently substituted by 1 to 5 groups selected from the group consisting of R^{a}" means that the phenyl or 5- to 10-membered heteroaryl may be substituted or unsubstituted. Among them, "the phenyl and 5- to 10-membered heteroaryl are each independently substituted by 1 to 5 groups selected from the group consisting of R^{a}" means that the phenyl is substituted by 1, 2, 3, 4 or 5 groups selected from the group consisting of R^{a}, or the 5- to 10-membered heteroaryl is substituted by 1, 2, 3, 4 or 5 groups selected from the group consisting of R^{a}. Moreover, when the phenyl or the 5- to 10-membered heteroaryl is substituted by 2, 3, 4 or 5 groups selected from the group consisting of R^{a}, the options of each R^{a} may be the same or different. Other similar definitions can be understood with reference to the foregoing content.

In the present invention, the expression "the phenyl, naphthyl, and 5- to 10-membered heteroaryl are each independently substituted by 2 adjacent groups, and the 2 adjacent groups and the carbon atoms to which they are respectively connected together form a 5- to 6-membered heterocyclic ring" means that the phenyl is substituted by two groups, and the two groups are in the adjacent positions, and the 2 adjacent groups and the carbon atoms to which they are respectively connected together form a 5- to 6-membered heterocyclic ring; or, the naphthyl is substituted by two groups, and the two groups are in the adjacent positions, and the 2 adjacent groups and the carbon atoms to which they are respectively connected together form a 5- to 6-membered heterocyclic ring; or, the 5- to 10-membered heteroaryl is substituted by two groups, and the two groups are in the adjacent positions, and the 2 adjacent groups and the carbon atoms to which they are respectively connected together form a 5- to 6-membered heterocyclic ring. Specifically, if the phenyl group is substituted by 2 adjacent groups, and the 2 adjacent groups and the carbon atoms to which they are respectively connected together form a 5- to 6-membered heterocyclic ring, and the 5- to 6-membered heterocyclic ring is 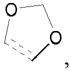 the formed group can be 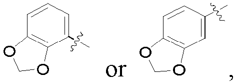 wherein the dotted double bond 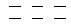 represents the fusion site of the heterocyclic ring and the phenyl. Other similar definitions can be understood with reference to the foregoing content.

From all the above descriptions, it is obvious to those skilled in the art that any group whose name is a composite name, for example "C1-C6 alkyl-O-C(=O)-", should refer to the conventional construction from the part derived therefrom from left to right, wherein "C1-C6 alkyl" is as defined above. For example, "C1-C6 alkyl-O-C(=O)-" can be 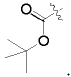 Other similar composite groups can be understood with reference to the foregoing content.

In the present invention, when L² is 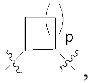 and p is 0, those skilled in the art can understand that the structural formula of L² is

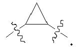

In the present invention, R^{4'} is selected from the group consisting of phenyl-(CH₂)ᵣ-, 5- to 10-membered heteroaryl-(CH₂)ᵣ-, and when r is 0, those skilled in the art can understand that R^{4'} is selected from the group consisting of phenyl, 5- to 10-membered heteroaryl. Other similar definitions can be understood with reference to the foregoing content.

In the present invention, the meaning of the structural formula 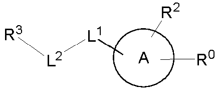 as shown in Formula I is that Ring A is substituted by three substituents, and the three substituents are R⁰, R² and 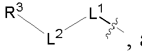 and the substitution positions and arrangement order of the three substituents can be adjusted arbitrarily. For example, in some embodiments, if Ring A is 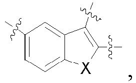 the structural formula as shown in Formula I can be Formula I-1, that is 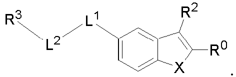 For another example, in some embodiments, if Ring A is 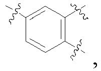 the structural formula as shown in Formula I can be Formula I-2, that is

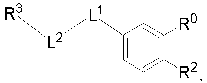

In the present invention, "treatment" generally refers to obtaining the desired pharmacological and/or physiological effects. The effect can be preventive based on the complete or partial prevention of a disease or its symptoms; and/or can be therapeutic based on partial or complete stabilization or cure of a disease and/or side effects caused by the disease. As used herein, "treatment" encompasses any treatment for a patient's disease, including: (a) preventing a disease or symptom from occurring in a patient who is susceptible to the disease or symptom but has not yet been diagnosed with the disease; (b) inhibiting a symptom of a disease, i.e., preventing its development; or (c) alleviating a symptom of a disease, i.e., causing the disease or symptom to regress.

In the present invention, "subject" refers to a vertebrate. In certain embodiments, the vertebrate refers to a mammal. The mammal includes, but is not limited to, livestock (e.g., cattle), pet (e.g., cat, dog, and horse), primate, mouse, and rat. In certain embodiments, the mammal refers to human.

In the present invention, "effective amount" refers to an amount that is effective at the required dose and time to achieve the desired therapeutic or preventive effect. The "therapeutically effective amount" of the substance/molecule of the present invention may vary according to factors such as the individual's disease state, age, gender, and weight, and the ability of the substance/molecule to elicit a desired response in the individual. The therapeutically effective amount also encompasses an amount in which the therapeutically beneficial effects of the substance/molecule outweigh any toxic or harmful consequences. "Prophylactically effective amount" refers to an amount that is effective at the required dose and time to achieve the desired preventive effect. Usually, but not necessarily, since the preventive dose is administered to a subject before the onset of a disease or in the early stage of the disease, the preventive effective amount will be lower than the therapeutic effective amount. In the case of cancer, the therapeutically effective amount of the drug can reduce the number of cancer cells; reduce the size of the tumor; inhibit (i.e., slow down to a certain extent, preferably stop) the infiltration of cancer cells into the surrounding organs; inhibit (i.e., slow down to a certain extent, preferably stop) tumor metastasis; inhibit tumor growth to a certain extent; and/or alleviate one or more symptoms related to cancer to a certain extent.

The pharmaceutical composition of the present invention may contain a pharmaceutically acceptable excipient, and the excipient includes but is not limited to: ion exchanger, aluminum oxide, aluminum stearate, lecithin, serum protein such as human albumin, buffer substance such as phosphate, glycerol, sorbic acid, potassium sorbate, mixture of partial glycerides of saturated vegetable fatty acids, water, salt or electrolyte, such as protamine sulfate, disodium hydrogen phosphate, potassium hydrogen phosphate, sodium chloride, zinc salt, colloidal silica, magnesium trisilicate, polyvinyl pyrrolidone, cellulosic substance, polyethylene glycol, sodium carboxymethyl cellulose, polyacrylate, beeswax, lanolin, etc.

The pharmaceutical composition of the present invention can be prepared into various forms according to different administration routes. For example, the pharmaceutical composition can be administered in any of the following ways: oral administration, spray inhalation, rectal administration, nasal administration, buccal administration, vaginal administration, topical administration, parenteral administration, such as subcutaneous, intravenous, intramuscular, intraperitoneal, intrathecal, intraventricular, intrasternal and intracranial injection or infusion, or by means of an explanted reservoir. Among them, oral or intravenous administration is preferred.

The compound of the present invention can optionally be used in combination with one or more other active ingredients, and their respective dosages and proportions can be adjusted by those skilled in the art according to specific symptoms, patient conditions and clinical needs.

As used herein, unless otherwise specified, the term "prodrug" refers to a derivative that can be hydrolyzed, oxidized or otherwise reacted in biological conditions (in vitro or in vivo) to provide the compound of the present invention. Prodrugs becomes an active compound only under biological conditions through the reaction, or they have no or only low activity in their unreactive form. Prodrugs can generally be prepared using well-known methods, such as those described in Burger's Medicinal Chemistry and Drug Discovery (1995) 172-178, 949-982 (Manfred E. Wolff, ed., 5th ed.).

A stereoisomer in the compound described herein, when specifically designated as (R)- or (S)-isomer in the chemical name, should be understood as the predominant configuration being the (R)-isomer or the (S)-isomer, respectively. Any asymmetric carbon atom may be present in the (R)-, (S)-, or (R, S)-configuration, preferably in the (R)- or (S)-configuration.

"Solvate" and "solvant" are used interchangeably, and refer to a compound that exists in combination with a solvent molecule. The combination may contain a stoichiometric amount of a solvent, for example, it can be a monohydrate or a dihydrate, or may contain any amount of water; for example, methanol or ethanol may form an "alcoholate", which may also be stoichiometric or non-stoichiometric. The term "solvate" as used herein refers to a solid form, i.e., although a compound in solvent of a solution can be solvated, it is not a solvate as described by the term used herein.

As used herein, the term "pharmaceutically acceptable salt" refers to (i) a salt formed by an acidic functional group (e.g., -COOH) present in a compound provided by the present invention and an appropriate inorganic or organic cation (base), which includes, but is not limited to, alkali metal salt, such as sodium salt, potassium salt, lithium salt, etc.; alkaline earth metal salt, such as calcium salt, magnesium salt, etc.; other metal salt, such as aluminum salt, iron salt, zinc salt, copper salt, nickel salt, cobalt salt, etc.; inorganic base salt, such as ammonium salt; organic base salt, such as tert-octylamine salt, dibenzylamine salt, morpholine salt, glucosamine salt, phenylglycine alkyl ester salt, ethylenediamine salt, *N-*methylglucosamine salt, guanidine salt, diethylamine salt, triethylamine salt, dicyclohexylamine salt, N,N'-dibenzylethylenediamine salt, chloroprocaine salt, procaine salt, diethanolamine salt, *N*-benzyl-phenethylamine salt, piperazine salt, tetramethylamine salt, tris(hydroxymethyl)aminomethane salt; and, (ii) a salt formed by an basic functional group (e.g. -NH₂) present in a compound provided by the present invention and an appropriate inorganic or organic anion (acid), which includes, but is not limited to, hydrohalide, such as hydrofluoride, hydrochloride, hydrobromide, hydroiodide, etc.; inorganic acid salt, such as nitrate, perchlorate, sulfate, phosphate, etc.; lower alkane sulfonate, such as methanesulfonate, trifluoromethanesulfonate, ethanesulfonate, etc.; aryl sulfonate, such as benzenesulfonate, p-toluenesulfonate, etc.; organic acid salt, such as acetate, malate, fumarate, succinate, citrate, tartrate, oxalate, maleate, etc.; amino acid salt, such as glycinate, trimethylglycinate, arginate, ornithinate, glutamate, aspartate, etc.

As used herein, the term "pharmaceutically acceptable ester" refers to an ester formed by -COOH present in a compound provided by the present invention and a suitable alcohol, or an ester formed by -OH present in a compound provided by the present invention and a suitable acid (e.g., carboxylic acid or oxygen-containing inorganic acid). Suitable ester groups include, but are not limited to, formate, acetate, propionate, butyrate, acrylate, ethylsuccinate, stearate or palmitate. Esters can undergo hydrolysis reaction in the presence of acid or base to generate corresponding acids or alcohols. In some embodiments, the ester formed by -COOH present in a compound provided by the present invention and a suitable alcohol is ethyl carboxylate, that is, the "pharmaceutically acceptable ester" is an ethyl ester.

As used herein, the term "crystal form" refers to a crystal structure of a substance. When a substance crystallizes, it is affected by various factors, which changes the intramolecular or intermolecular bonding mode, causing molecules or atoms to be arranged differently in the lattice space, forming different crystal structures. The compound of the present invention can exist in one crystal structure or in multiple crystal structures, that is, it has "polymorphism". The compound of the present invention may exist in different crystalline forms.

As used herein, the term "modulator" refers to a molecule that can interact directly or indirectly with a target. The modulator includes, but is not limited to, agonist, partial agonist, inverse agonist, and antagonist. In some embodiments of the present application, the modulator is an agonist.

As used herein, the term "agonist" refers to a molecule that binds to a specific receptor and triggers a reaction in a cell. Agonist can mimic the effects of an endogenous ligand (e.g., LPA, prostaglandin, hormone, or neurotransmitter) that binds to the same receptor.

As used herein, the term "antagonist" refers to a molecule that weakens, inhibits, or prevents the action of another molecule or the activity of a receptor site. Antagonist includes, but is not limited to, competitive antagonist, non-competitive antagonist, uncompetitive antagonist, partial agonist, and inverse agonist.

The present invention is further explained below in conjunction with specific examples. Unless otherwise specified, the raw materials used in the following examples could be purchased commercially.

### Example 1

### Step 1: Ethyl 2-(4-acetylamino-2-acetylphenoxy)acetate

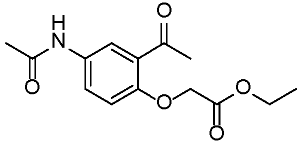

N-(3-acetyl-4-hydroxyphenyl)acetamide (1.01 g, 5.23 mmol), ethyl bromoacetate (1 mL, 8.98 mmol), potassium carbonate (1.33 g, 9.62 mmol) and potassium iodide (0.16 g, 0.97 mmol) were added to a pear-shaped bottle, added with acetone (30 mL), heated to reflux at 60 °C, and reacted for 2 h; when TLC monitoring indicated that the reaction was completed, an appropriate amount of water was added; the system was extracted with dichloromethane 3 times; the organic phases were combined, dried over anhydrous sodium sulfate, filtered and concentrated, and purified by silica gel column chromatography (DCM/MeOH = 30:1) to obtain 1.32 g of white solid with a yield of 90.4%.

### Step 2: Ethyl 5-acetylamino-3-methylbenzofuran-2-carboxylate

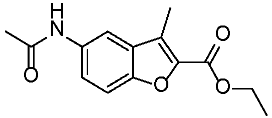

Ethyl 2-(4-acetylamino-2-acetylphenoxy)acetate (1.32 g, 4.72 mmol), potassium carbonate (0.85 g, 6.15 mmol) and DMF (41 mL) were added to a pear-shaped bottle, heated to reflux at 140°C, and reacted for 1 h; when TLC monitoring indicated that the reaction was completed, a small amount of EA was added; the system was filtered with diatomaceous earth; the filtrate was added with saturated sodium chloride solution, extracted with EA 3 times; the organic phases were combined, and washed twice with saturated sodium chloride solution; the organic phase was dried over anhydrous sodium sulfate, filtered and concentrated, and purified by silica gel column chromatography (DCM/MeOH = 50:1) to obtain 0.78 g of white solid with a yield of 63.6%.

### Step 3: Ethyl 5-amino-3-methylbenzofuran-2-carboxylate

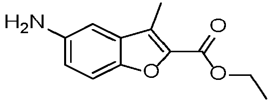

Ethyl 5-acetylamino-3-methylbenzofuran-2-carboxylate (0.78 g, 2.79 mmol) was dissolved in ethanol (16 mL), added with 3N HCl (16 mL), heated to reflux at 80°C, and reacted for 2 h; when TLC monitoring indicated that the reaction was completed, saturated sodium bicarbonate solution was added to adjust the pH to 7 to 8; EA was used to perform extraction 3 times; the organic phases were combined and dried over anhydrous sodium sulfate, filtered and concentrated, and purified by silica gel column chromatography (DCM/MeOH = 30: 1) to obtain 0.44 g of light-yellow solid with a yield of 71.2%.

### Step 4: Ethyl 3-methyl-5-((2-phenylethyl)sulfonyl)benzofuran-2-carboxylate

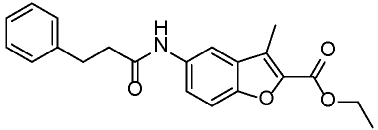

Phenylpropionic acid (0.08 g, 0.53 mmol) was dissolved in DMF (2 mL), added with HATU (0.26 g, 0.68 mmol) and DIPEA (0.1 mL), stirred at room temperature for 10 min, then added with ethyl 5-amino-3-methylbenzofuran-2-carboxylate (0.10 g, 0.46 mmol), and stirred at room temperature for 1 h, until TLC monitoring indicated that the reaction was completed. Saturated sodium chloride solution was added, and EA was used to perform extraction twice. The organic phases were combined and washed once with saturated sodium chloride solution. The organic phase was dried over anhydrous sodium sulfate, filtered and concentrated, and purified by silica gel column chromatography (DCM/MeOH = 100:1) to obtain 188 mg of a light-yellow solid with a yield of 95.4%. ¹H NMR (400 MHz, CDCl₃) δ 8.14 (s, 1H), 7.96 (s, 1H), 7.38 - 7.13 (m, 7H), 4.43 (q, *J =* 7.2 Hz, 2H), 3.05 (t, *J =* 7.6 Hz, 2H), 2.70 (t, *J =* 7.6 Hz, 2H), 2.45 (s, 3H), 1.43 (t, *J* = 7.2 Hz, 3H). ¹³C NMR (101 MHz, CDCl₃) δ 171.11, 150.53, 151.21, 141.60, 140.61, 133.54, 129.27, 128.59, 128.32, 126.35, 125.74, 121.50, 112.73, 112.11, 61.25, 39.05, 31.60, 14.38, 9.36.

### Example 2: Ethyl 5-cinnamamido-3-methylbenzofuran-2-carboxylate

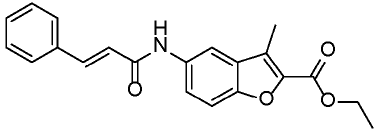

The preparation method was the same as that of Example 1, except that cinnamic acid was used instead of phenylpropionic acid to obtain the title compound. 236 mg of light-white solid was obtained, with a yield of 98.7%. ¹H NMR (400 MHz, CDCl₃) δ 8.85 (s, 1H), 8.15 (s, 1H), 7.77 (d, *J =* 15.6 Hz, 1H), 7.52 - 7.18 (m, 7H), 6.76 (d, *J =* 15.6 Hz, 1H), 4.44 (q, *J =* 7.2 Hz, 2H), 2.41 (s, 3H), 1.43 (t, *J* = 7.2 Hz, 3H). ¹³C NMR (101 MHz, CDCl₃) δ 164.87, 160.50, 151.30, 142.20, 141.61, 134.53, 133.84, 129.9 4, 129.37, 128.79, 127.87, 125.78, 121.62, 121.03, 112.84, 112.22, 61.25, 14.38, 9.32.

### Example 3: Ethyl (E)-5-(3-(3-iodophenyl)acrylamido)-3-methylbenzofuran-2-carboxylate

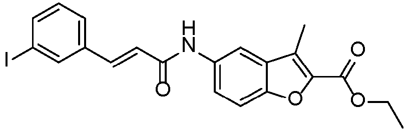

The preparation method was the same as that of Example 1, except that (E)-3-(3-iodophenyl)acrylic acid was used instead of phenylpropionic acid to obtain the title compound. 120 mg of white solid was obtained, with a yield of 70.2%. ¹H NMR (400 MHz, CDCl₃) δ 8.18 (s, 1H), 8.14 (s, 1H), 7.83 (s, 1H), 7.72 - 7.58 (m, 2H), 7.47 - 7.36 (m, 3H), 7.06 (t, *J =* 7.8 Hz, 1H), 6.62 (d, *J =* 15.5 Hz, 1H), 4.44 (q, *J =* 7.2 Hz, 2H), 2.51 (s, 3H), 1.44 (t, *J =* 7.2 Hz, 3H).

### Example 4: Ethyl 3-methyl-5-((1S,2R)-2-phenylcyclopropane-1-carboxamide) benzofuran-2-carboxylate

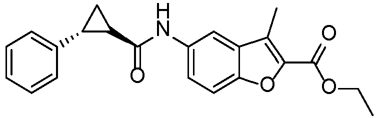

The preparation method was the same as that of Example 1, except that (1S,2R)-2-phenylcyclopropane-1-carboxylic acid was used instead of phenylpropionic acid to obtain the title compound. 151 mg of white solid was obtained, with a yield of 55.5%. ¹H NMR (400 MHz, CDCl₃) δ 8.16 (s, 1H), 8.09 (s, 1H), 7.43 - 7.17 (m, 5H), 7.09 (d, *J =* 7.6 Hz, 2H), 4.43 (q, *J =* 7.2 Hz, 2H), 2.64 - 2.56 (m, 1 H), 2.50 (s, 3H), 1.91 - 1.83 (m, 1H), 1.78 - 1.70 (m, 1H), 1.44 (t, *J =* 7.2 Hz, 3H), 1.39 -1.30 (m, 1H). ¹³C NMR (101 MHz, CDCl₃) δ 170.70, 16 0.53, 151.15, 141.66, 140.43, 133.77, 129.41, 128.51, 126.44, 126.02, 121.05, 112.33, 112.23, 61.23, 27.42, 25.92, 16.45, 14.38, 9.39.

### Example 5: Ethyl 3-methyl-5-(4-oxo-4-phenylbutylamino)benzofuran-2-carboxylate

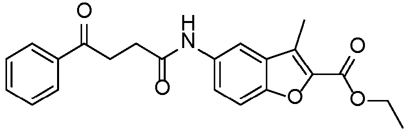

The preparation method was the same as that of Example 1, except that 4-oxo-4-phenylbutyric acid was used instead of phenylpropionic acid to obtain the title compound. 231 mg of yellow solid is obtained, with a yield of 91.3%. ¹H NMR (400 MHz, CDCl₃) δ 8.50 (s, 1H), 8.01 (m, 3H), 7.66 - 7.26 (m, 5H), 4.54 - 4.38 (m, 2H), 3.59 - 3.41 (m, 2H), 2.98 - 2.82 (m, 2H), 2.48 (d, *J =* 12.0 Hz, 3H), 1.45 (m, 3H). ¹³C NMR (101 MHz, CDCl₃) δ 199.55, 170.69, 160.44, 151.05, 141.55, 136.33, 133.80, 133.55, 129.24, 128.69, 128.13, 125.76, 121.00, 112.11, 112.04, 61.14, 33.99, 31.13, 14.38, 9.34.

### Example 6: Ethyl 3-methyl-5-(5-phenylpentanamido)benzofuran-2-carboxylate

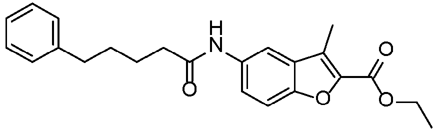

The preparation method was the same as that of Example 1, except that 4-oxo-4-phenylbutyric acid was used instead of phenylpropionic acid to obtain the title compound. 0.2 g of white solid was obtained, with a yield of 72.2%. ¹H NMR (400 MHz, CDCl₃) δ 8.82 (s, 1H), 8.02 (d, *J =* 6.0 Hz, 1H), 7.42 - 7.04 (m, 7H), 4.43 (q, *J =* 7.2 Hz, 2H), 2.59 (dd, *J =* 13.2, 5.6 Hz, 2H), 2.50 - 2.35 (m, 5H), 1.86 - 1.60 (m, 4H), 1.43 (t, *J =* 7.2 Hz, 3H). ¹³C NMR (101 MHz, CDCl₃) δ 172.35, 160.56, 151.17, 142.01, 141.55, 133.9 2, 129.21, 128.32, 125.81, 125.71, 121.71, 112.74, 112.00, 61.26, 37.16, 35.67, 31.05, 25.41, 14.39, 9.34.

### Example 7: Ethyl 3-methyl-5-(3-phenylpropoxy)benzofuran-2-carboxylate

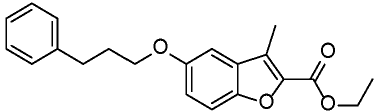

Ethyl 5-hydroxy-3-methylbenzofuran-2-carboxylate (189 mg, 0.86 mmol), triphenylphosphine (458 mg, 1.74 mmol), 3-phenylpropanol (175 mg, 1.29 mmol), diisopropyl azodicarboxylate (347 mg, 1.72 mmol) were dissolved in anhydrous tetrahydrofuran (10 mL) and reacted at room temperature in argon for 4 h. After TLC monitoring indicated that the reaction was completed, water (10 mL) was added and ethyl acetate (30 mL) was used to perform extraction three times. The organic phase was washed with saturated aqueous sodium chloride solution and dried over anhydrous sodium sulfate. The crude product was separated by column chromatography (PE/EA = 15/1 10/1) to obtain 0.15 g of a yellow solid with a yield of 54%. ¹H NMR (400 MHz, CDCl₃) δ 7.43 (d, *J =* 9.0 Hz, 1H), 7.33 - 7.16 (m, 5H), 7.06 (dd, *J* = 9.0, 2.6 Hz, 1H), 6.97 (d, *J =* 2.6 Hz, 1H), 4.44 (q, *J =* 7*.*1 Hz, 2H), 4.01 (t, *J =* 6.2 Hz, 2H), 2.84 (t, *J =* 7.6 Hz, 2H), 2.54 (s, 3H), 2.20 - 2.08 (m, 2H), 1.44 (t, *J =* 7.1 Hz, 3H). ¹³C NMR (101 MHz, CDCl₃) δ 160.6, 155.7, 149.6, 141.7, 129.6, 128.7, 128.6, 126.1, 125.7, 118.1, 113.0, 103.4, 67.8, 61.2, 32.3, 31.0, 14.5, 9.6. HRMS (ESI) [M+H]⁺ Calculated value for C₂₁H₂₃O₄: 339.1596; Measured value: 339.1592.

### Example 8

### Step 1: Ethyl 3-methylbenzofuran-2-carboxylate

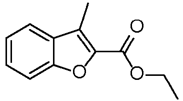

2-Hydroxyacetophenone (2.72 g, 20 mmol), ethyl bromoacetate (5.01 g, 30 mmol), and potassium carbonate (5.52 g, 40 mmol) were added to N,N-dimethylformamide (25 mL), heated to 160 °C and reacted for 3 h; after TLC monitoring indicated that the reaction was completed, the reaction mixture was cooled to room temperature, and the solid was obtained by filtration under reduced pressure. The filter cake was rinsed three times with ethyl acetate (25 mL). The filtrate was combined, and the solvent was removed by distillation under reduced pressure, the residue was added with water (100 mL) for dissolution and extracted with ether (100 mL) three times. The organic phase was washed with saturated sodium chloride aqueous solution and dried over anhydrous sodium sulfate. The crude product was separated by column chromatography (PE/EA = 15/1 ~ 10/1) to obtain 1.54 g of white solid, with a yield of 38%.

### Step 2: Ethyl 3-methyl-5-(chlorosulfonyl)benzofuran-2-carboxylate

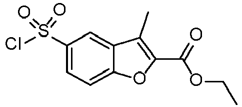

Ethyl 3-methylbenzofuran-2-carboxylate (3.06 g, 15 mmol) was added to chloroform (45 mL), stirred at 0 °C for 10 minutes, added dropwise with chlorosulfonic acid (8.70 g, 75 mmol) in chloroform (45 mL), stirred at room temperature for 4 h. After TLC monitoring indicated that the reaction was completed, the reaction solution was poured into ice water and extracted three times with dichloromethane (150 mL). The organic phases were combined and washed with saturated sodium chloride aqueous solution, dried over anhydrous sodium sulfate, and the crude product was recrystallized from petroleum ether/ethyl acetate to obtain 2.07 g of white solid with a yield of 46%.

### Step 3: Ethyl 3-methyl-5-(N-phenylethylsulfamoyl)benzofuran-2-carboxylate

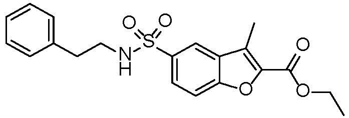

Ethyl 5-(chlorosulfonyl)-3-methylbenzofuran-2-carboxylate (2.07 g, 6.86 mmol) and potassium carbonate (1.89 g, 13.72 mmol) were dissolved in anhydrous dichloromethane (80 mL), added dropwise with phenylethylamine (0.97 g, 8.23 mmol) in anhydrous dichloromethane (20 mL) at room temperature, and stirred at room temperature for 3 h. After TLC monitoring indicated that the reaction was completed, the solvent was removed by distillation under reduced pressure, the residue was dissolved with water (100 mL) and extracted three times with ethyl acetate (100 mL). The organic phase was washed with saturated sodium chloride aqueous solution and dried over anhydrous sodium sulfate. The crude product was separated by column chromatography (PE/EA = 10/1~ 5/1) to obtain 2.33 g of a white solid with a yield of 88%. ¹H NMR (400 MHz, CDCl₃) δ 8.15 (d, *J =* 1.8 Hz, 1H), 7.84 (dd, *J =* 8.8, 1.9 Hz, 1H), 7.59 (d, *J =* 8.8 Hz, 1H), 7.25 - 7.13 (m, 3H), 7.10 - 6.99 (m, 2H), 4.77 (t, *J =* 6.2 Hz, 1H), 4.47 (q, *J =* 7.1 Hz, 2H), 3.24 (q, *J =* 6.9 Hz, 2H), 2.77 (t, *J =* 7.0 Hz, 2H), 2.59 (s, 3H), 1.45 (t, *J =* 7.1 Hz, 3 H). ¹³C NMR (101 MHz, CDCl₃) δ 159.9, 155.8, 143.0, 137.6, 135.2, 129.4, 128.7, 126.8, 126.2, 125.6, 121.6, 113.0, 61.6, 44.3, 35.8, 14.4, 9.4. HRMS (ESI) [M+H]⁺ Calculated value for C₂₀H₂₂NO₅S: 388.1219; Measured value: 388.1213.

### Step 4: Ethyl 3-methyl-5-(N-benzyl-N-phenylethylsulfamoyl)benzofuran-2-carboxylate

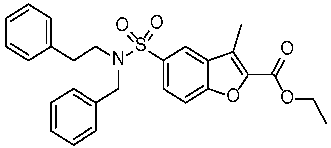

Ethyl 3-methyl-5-(*N*-phenylethylsulfamoyl)benzofuran-2-carboxylate (195 mg, 0.5 mmol), benzyl bromide (171 mg, 1.0 mmol), and potassium carbonate (276 mg, 2.0 mmol) were dissolved in anhydrous dichloromethane (10 mL), and stirred at 60 °C overnight. After TLC monitoring indicated that the reaction was completed, the solvent was removed by distillation under reduced pressure; the residue was dissolved with water (20 mL) and extracted three times with dichloromethane (20 mL) ; the organic phase was washed with saturated sodium chloride aqueous solution, and dried over anhydrous sodium sulfate. The crude product was separated by column chromatography (PE/EA = 20/1 ~ 2/1) to obtain 229 mg of a white solid with a yield of 96%. ¹H NMR (400 MHz, CDCl₃) δ 8.14 (d, *J =* 1.5 Hz, 1H), 7.87 (dd, *J =* 8.8, 1.7 Hz, 1H), 7.63 (d, *J =* 8.8 Hz, 1H), 7.39 - 7.23 (m, 5H), 7.22 - 7.09 (m, 3H), 6.95 (d, *J =* 6.8 Hz, 2H), 4.48 (q, *J =* 7.1 Hz, 2H), 4.40 (s, 2H), 3.45 - 3.28 (m, 2H), 2.71 - 2.62 (m, 2H), 2.60 (s, 3H), 1.46 (t, *J=* 7.1 Hz, 3H). ¹³C NMR (101 MHz, CDCl₃) δ 159.9, 155.8, 143.0, 138.3, 136.0, 135.6, 129.4, 128.7, 128.7, 128.5, 128.5, 128.0, 126.5, 126.2, 125.6, 121.5, 113.1, 61.6, 52.2, 49.5, 35.3, 14.4, 9.4. HRMS (ESI) [M+H]⁺ Calculated value for C₂₇H₂₈NO₅S: 478.1688; Measured value: 478.1680.

### Example 9: Ethyl 3-methyl-5-(N-(4-bromobenzyl)-N-phenylethylsulfamoyl)benzofuran-2- carboxylate

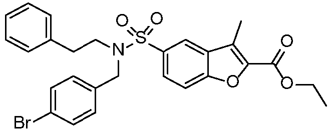

The preparation method was the same as Example 8, except that 4-bromobenzyl bromide was used instead of benzyl bromide to obtain the title compound. 193 mg of white solid was obtained, with a yield of 70%. ¹H NMR (400 MHz, CDCl₃) δ 8.14 (d, *J* = 1.8 Hz, 1H), 7.87 (dd, *J* = 8.8, 1.9 Hz, 1H), 7.64 (d, *J* = 8.7 Hz, 1H), 7.42 (d, *J =* 8.3 Hz, 2H), 7.17 (dd, *J* = 1 7.6, 7.7 Hz, 5H), 6.96 (d, *J =* 6.5 Hz, 2H), 4.49 (q, *J =* 7.1 Hz, 2H), 4.34 (s, 2H), 3.48 - 3.30 (m, 2H), 2.70 - 2.64 (m, 2H), 2.62 (s, 3H), 1.47 (t, *J =* 7.1 Hz, 3H). ¹³C NMR (101 MHz, CDCl₃) δ 159.8, 155.8, 143.0, 138.1, 135.3, 135.2, 131.8, 130.0, 129.4, 128.6, 128.5, 126.6, 126.2, 125.5, 121.9, 121.5, 113.1, 61.6, 51.6, 49.6, 35.2, 14.4, 9.4.

### Example 10: Ethyl 3-methyl-5-(N-(4-(tert-butyloxycarbonyl)benzyl)-N-phenylethylsulfamoyl)benzofuran-2-carboxylate

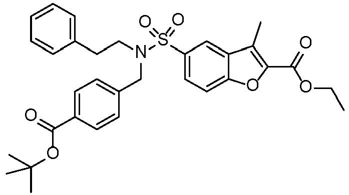

The preparation method was the same as that of Example 8, except that 4-(tert-butyloxycarbonyl)benzyl bromide was used instead of benzyl bromide to prepare the title compound. 273 mg of white solid was obtained, with a yield of 94%. ¹H NMR (400 MHz, CDCl₃) δ 8.12 (d, *J =* 1.6 Hz, 1H), 7.92 (d, *J =* 8.2 Hz, 2H), 7.87 (dd, *J=* 8.8, 1.8 Hz, 1H), 7.62 (d, *J=* 8.8 Hz, 1H), 7.31 (d, *J =* 8 (s, 9H), 1.45 (t, *J= 7.1* Hz, 3H). ¹³C NMR (101 MHz, CDCl₃) δ 165.3, 159.8, 155.8, 143.0, 140.8, 138.1, 135.3, 131.7, 129.7, 129.4, 128.6, 128.5, 128.1, 126.5, 126.2, 125.5, 121.5, 113.1, 81.1, 61.5, 51.9, 49.8, 35.2, 28.2, 14.4, 9.4.

### Example 11: Ethyl 3-methyl-5-(N-(quinolin-8-ylmethyl)-N-phenethylsulfamoyl) benzofuran-2-carboxylate

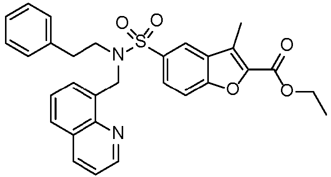

The preparation method was the same as Example 8, except that 8-(bromomethyl)quinoline was used instead of benzyl bromide to obtain the title compound. 222 mg of white solid was obtained, with a yield of 84%. ¹H NMR (400 MHz, CDCl₃) δ 8.83 (d, *J* = 2.6 Hz, 1H), 8.19 - 8.07 (m, 2H), 7.95 (d, *J =* 7.1 Hz, 1H), 7.88 (d, *J =* 10.3 Hz, 1H), 7.71 (d, *J =* 8.0 Hz, 1 H), 7.61 - 7.47 (m, 2H), 7.36 (dd, *J =* 8.2, 4.2 Hz, 1H), 7.11 (dq, *J =* 14.2, 7.0 Hz, 3H), 6.94 (d, *J =* 7.0 Hz, 2H), 5.22 (s, 2H), 4.46 (q, *J =* 7.1 Hz, 2H), 3.71 - 3.50 (m, 2H), 2.91 - 2.66 (m, 2H), 2.56 (s, 3H), 1.44 (t, *J =* 7.1 Hz, 3H). ¹³C NMR (101 MHz, CDCl₃) δ 159.9, 155.6, 149.5, δ 146. 2, 142.8, 138.4, 136.4, 135.7, 134.8, 129.7, 129.2, 128.7, 128.4, 128.1, 127.7, 126.5, 126.3, 126.3, 125.6, 121.4, 121.2, 11 2.8, 61.5, 50.8, 47.5, 35.3, 14.4, 9.4. HRMS (ESI) [M+H]⁺ Calculated value for C₃₀H₂₉N₂O₅S: 529.1797; Measured value: 529.1790.

### Example 12: Ethyl 3-methyl-5-(N-phenyl-N-phenethylsulfamoyl)benzofuran-2-carboxylate

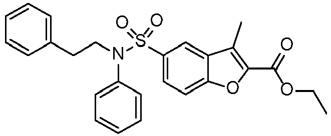

Ethyl 3-methyl-5-(chlorosulfonyl)benzofuran-2-carboxylate (91 mg, 0.3 mmol), *N-*phenethylaniline (59 mg, 0.3 mmol), and potassium carbonate (46 mg, 0.33 mmol) were dissolved in anhydrous dichloromethane (10 mL) and stirred at 60 °C overnight. After TLC monitoring indicated that the reaction was completed, the solvent was removed by distillation under reduced pressure; the residue was dissolved in water (10 mL), and extracted with dichloromethane (20 mL) three times. The organic phase was washed with saturated aqueous sodium chloride solution and dried over anhydrous sodium sulfate. The crude product was separated by column chromatography (PE/EA = 10/1 ~ 5/1) to obtain 90 mg of a yellow solid with a yield of 65%. ¹H NMR (400 MHz, CDCl₃) δ 7.88 (s, 1H), 7.63 (d, *J =* 8.8 Hz, 1H), 7.56 (d, *J =* 8.7 Hz, 1H), 7.39 - 7.31 (m, 3H), 7.30 - 7.24 (m, 2H), 7.23 - 7.1 8 (m, 1H), 7.17 - 7.10 (m, 2H), 7.09 - 7.00 (m, 2H), 4.48 (q, *J =* 7.1 Hz, 2H), 3.90 - 3.77 (m, 2H), 2.89 - 2.76 (m, 2H), 2.54 (s, 3H), 1.4 6 (t, *J= 7.1* Hz, 3H). ¹³C NMR (101 MHz, CDCl₃) δ 159.9, 155.9, 142.9, 139.0, 138.0, 133.6, 129.2, 128.9, 128.8, 128.5, 128.2, 126.8, 126.6, 125. 6, 122.0, 112.6, 61.5, 52.2, 35.2, 14.4, 9.3. HRMS (ESI) [M+H]⁺ Calculated value for C₂₆H₂₆NO₅S: 464.1532; Measured value: 464.1525.

### Example 13: Ethyl 3-methyl-5-(N,N-diphenylethylaminosulfonyl)benzofuran-2-carboxylate

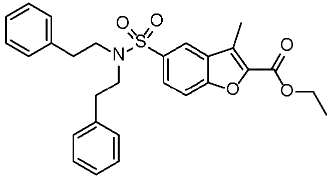

The preparation method was the same as that of Example 12, except that diphenylethylamine was used instead of *N*-phenylethylaniline to obtain the title compound. 112 mg of white oil was obtained, with a yield of 76%. ¹H NMR (400 MHz, CDCl₃) δ 8.16 (s, 1H), 7.84 (d, *J =* 8.7 Hz, 1H), 7.60 (d, *J =* 8.7 Hz, 1H), 7.29 (t, *J =* 7.2 Hz, 4H), 7.22 (t, *J =* 7.3 Hz, 2H), 7.1 6 (d, *J =* 7.0 Hz, 4H), 4.49 (q, *J =* 7.1 Hz, 2H), 3.56 - 3.40 (m, 4H), 2.96 - 2.82 (m, 4H), 2.63 (s, 3H), 1.48 (t, *J =* 7.1 Hz, 3H).¹³C NMR MHz, CDCl₃) δ 159.9, 155.7, 142.9, 138.4, 135.4, 129.4, 128.8, 128.6, 126.6, 126.3, 125.6, 121.5, 113.0, 61.6, 50.0, 35.6, 14.4, 9.4. HRMS (ESI) [M+H]⁺ Calculated value for C₂₈H₃₀NO₅S: 492.1845; Measured value: 492.1836.

### Example 14

### Step 1: 4-(4-(Methylsulfonyl)piperazin-1-yl)-N-phenylethylaniline

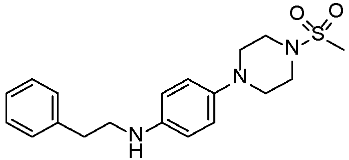

4-(4-(Methylsulfonyl)piperazin-1-yl)aniline (0.46 g, 1.8 mmol), 2-iodoethylbenzene (0.28 g, 1.2 mmol), and potassium carbonate (0.25 g, 2.7 mmol) were dissolved in acetonitrile (8 mL), heated to 60 °C and reacted for 24 h. After TLC monitoring indicated that the reaction was completed, the reaction mixture was cooled to room temperature; the solvent was removed by distillation under reduced pressure; the residual was added with water (10 mL) and extracted three times with ethyl acetate (10 mL). The organic phase was washed with saturated sodium chloride aqueous solution and dried over anhydrous sodium sulfate. The crude product was separated by column chromatography to obtain 155 mg of a yellow solid with a yield of 36%. ¹H NMR (400 MHz, CDCl₃) δ 7.26 (dt, *J =* 35.0, 7.5 Hz, 5H), 6.83 (d, *J =* 7.5 Hz, 2H), 6.57 (d, *J =* 8.3 Hz, 2H), 3.54 - 3.23 (m, 7H), 3.09 (s, 4H), 2.88 (t, *J =* 6.9 Hz, 2H), 2.79 (s, 3H). ¹³C NMR (101 MHz, CDCl₃) δ 143.4, 142.8, 139.4, 128.8, 128.6, 126.5, 119.7, 114.0, 51.1, 46.1, 4 5.7, 35.6, 34.2.

### Step 2: Ethyl 3-methyl-5-(N-(4-(4-(methylsulfonyl)piperazin-1-yl)phenyl)- N-phenylethylsulfamoyl)benzofuran-2-carboxylate

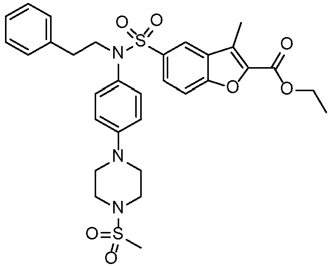

The preparation method was the same as that of Example 12, except that diphenylethylamine was used instead of *N*-phenylethylaniline to obtain the title compound. 275 mg of yellow oil was obtained, with a yield of 87%. ¹H NMR (400 MHz, CDCl₃) δ 7.87 (d, *J =* 1.8 Hz, 1H), 7.58 (dd, *J =* 8.8, 1.9 Hz, 1H), 7.50 (d, *J =* 8.8 Hz, 1H), 7.20 (t, *J =* 7.2 Hz, 2H), 7.13 (dd, *J =* 8.6, 5.9 Hz, 1H), 7.07 (d, *J =* 6.9 Hz, 2H), 6.89 (d, *J =* 9.0 Hz, 2H), 6.81 (d, *J =* 9.0 Hz, 2H), 4.41 (q, *J =* 7.1 Hz, 2H), 3.79 - 3.69 (m, 2H), 3.39 - 3.31 (m, 4H), 3.30 - 3.22 (m, 4H), 2.80 (s, 3H), 2.77 - 2.68 (m, 2H), 2.50 (s, 3H), 1.40 (t, *J =* 7.1 Hz, 3H). ¹³C NMR (101 MHz, CDCl₃) δ 159.8, 15 5.8, 150.2, 142.8, 138.1, 133.8, 130.9, 129.9, 129.1, 128.8, 128.5, 126.9, 126.5, 125.5, 122.0, 116.6, 112.6, 61.5, 52.2, 48.7, 45.7, 35.0, 34.5, 14.4, 9.4.

### Example 15

### Step 1: N-phenethyl-4-phenoxyaniline

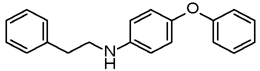

Phenylacetaldehyde (0.06 g, 0.5 mmol), 4-aminodiphenyl ether (0.09 g, 0.5 mmol), and trimethyl orthoformate (85 mg, 0.8 mmol) were dissolved in methanol (10 mL) and stirred at 0 °C; sodium cyanoborohydride (0.04 g, 0.6 mmol) was added in batches, and reacted at room temperature for 10 h. After TLC monitoring indicated that the reaction was completed, the solvent was removed by distillation under reduced pressure. The residual was added with water (10 mL) and extracted three times with ethyl acetate (10 mL). The organic phase was washed with saturated sodium chloride aqueous solution and dried over anhydrous sodium sulfate. The crude product was separated by column chromatography to obtain a yellow solid 140 mg, yield 65%.

### Step 2: Ethyl 3-methyl-5-(N-(4-phenoxyphenyl)-N-phenethylsulfamoyl) benzofuran-2-carboxylate

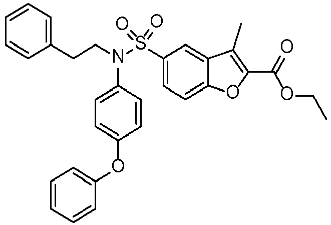

The preparation method was the same as Example 12, except that N-phenethyl-4-phenoxyaniline was used instead of *N*-phenethylaniline to obtain the title compound. 123 mg of white solid was obtained, with a yield of 44%. ¹H NMR (400 MHz, CDCl₃) δ 7.92 (d, *J =* 1.9 Hz, 1H), 7.67 (dd, *J =* 8.8, 1.9 Hz, 1H), 7.58 (d, *J =* 8.8 Hz, 1H), 7.39 (t, *J =* 7.9 Hz, 2H), 7.28 (t, *J=* 7.2 Hz, 2H), 7.18 (dt, *J =* 22.1, 7.0 Hz, 4H), 7.07 (t, *J =* 7.8 Hz, 2H), 7.02 - 6.97 (m, 2H), 6.93 (d, *J =* 9.0 Hz, 2H), 4.49 (q, *J =* 7.1 Hz, 2H), 3.93 - 3.65 (m, 2H), 2.94 - 2.79 (m, 2H), 2.57 (s, 3H), 1.47 (t, *J= 7.1* Hz, 3H). ¹³C NMR (101 MHz, CDCl₃) δ 159.9, 157.3, 156.3, 155.9, 142.9, 1 38.0, 133.6, 133.6, 130.4, 129.9, 129.2, 128.8, 128.5, 126.8, 126.6, 125.6, 124.1, 122.0, 119.5, 118.6, 112.7, 61.6, 52.3, 35.2, 14.4, 9.3.

### Example 16: 3-Methyl-5-(3-phenylpropylamino)benzofuran-2-carboxylic acid (GPR132-A-1)

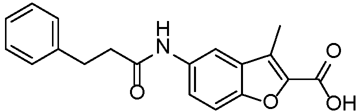

Ethyl 3-methyl-5-(3-phenylpropylamino)benzofuran-2-carboxylate (0.19 g, 0.53 mmol) was dissolved in ethanol (8 mL), added with sodium hydroxide solution (0.11 g, 2.67 mmol, dissolved in 2 mL water), heated to reflux and reacted for 2 h. After the reaction was completed, the ethanol was removed by concentration. After an appropriate amount of water was added for dilution, 3N HCl was added to adjust the pH to 2 to 3, and a large amount of white solid was precipitated. Filtration was performed and the filter cake was washed with distilled water, and dried to obtain 148.7 mg of a light-yellow solid with a yield of 85.9%. ¹H NMR (400 MHz, DMSO-*d₆*) δ 13.40 (s, 1H), 10.06 (s, 1H), 8.10 (d, *J =* 2.1 Hz, 1H), 7.59 - 7.49 (m, 2H), 7.34 - 7.24 (m, 4H), 7.22 - 7.15 (m, 1H), 2.93 (t, *J =* 8.0 Hz, 2H), 2.65 (t, *J =* 8.0 Hz, 2H), 2.49 (s, 3H). ¹³C NMR (101 MHz, DMSO-*d₆*) δ 170.81, 161.51, 150.27, 142.28, 141.64, 135.52, 129.12, 128.81, 128.71, 126.42, 124.84, 120.96, 112.41, 111.10, 38.35, 31.30, 9.58. HRMS (ESI) [M-H]⁻ Calculated value for C₁₉H₁₆NO₄S: 322.1085; Measured value: 322.1078.

### Example 17: 5-Cinnamylamino-3-methylbenzofuran-2-carboxylic acid (GPR132-A-2)

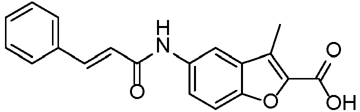

The preparation method was the same as that of Example 16, except that ethyl 5-cinnamylamino-3-methylbenzofuran-2-carboxylate was used instead of ethyl 3-methyl-5-(3-phenylpropylamino)benzofuran-2-carboxylate to obtain the title compound. The obtained product was a light-yellow solid (155 mg), with a yield of 71.4%. ¹H NMR (400 MHz, DMSO-*d₆*) δ 13.46 (s, 1H), 10.37 (s, 1H), 8.23 (d, *J =* 2.0 Hz, 1H), 7.69 - 7.61 (m, 4H), 7.60 (d, *J =* 5.6 Hz, 1H), 7.49 - 7.37 ( m, 3H), 6.86 (d, *J =* 15.6 Hz, 1H), 2.52 (s, 3H). ¹³C NMR (101 MHz, DMSO-*d₆*) δ 163.98, 161.52, 150.46, 142.44, 140.59, 135.56, 135.17, 130.27, 129.51, 129.25, 128.20, 124.82, 122.68, 121.04, 112.57, 111.31, 9.61. HRMS (ESI) [M-H]⁻ Calculated value for C₁₀H₁₄NO₄S: 320.0928; Measured value: 320.0920.

### Example 18: (E)-5-(3-(3-iodophenyl)acrylamido)-3-methylbenzofuran-2-carboxylic acid (GPR132-A-3)

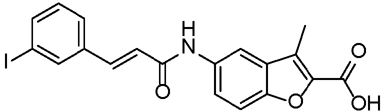

The preparation method was the same as that of Example 16, except that ethyl (E)-5-(3-(3-iodophenyl)acrylamido)-3-methylbenzofuran-2-carboxylate was used instead of ethyl 3-methyl-5-(3-phenylpropylamino)benzofuran-2-carboxylate to obtain the title compound. 94 mg of white solid was obtained with a yield of 83.2%. ¹H NMR (400 MHz, DMSO-*d₆*) δ 13.41 (s, 1H), 10.34 (s, 1H), 8.21 (s, 1H), 7.99 (s, 1H), 7.82 - 7.44 (m, 5H), 7.22 (t, *J =* 7.8 Hz, 1H), 6.86 (d, *J =* 15.7 Hz, 1H), 2.50 (s, 3H). ¹³C NMR (101 MHz, DMSO-*d₆*) δ 163.60, 161.48, 150.48, 142.37, 138.80, 138.54, 137.61, 136.43, 135.45, 13 1.48, 129.23, 127.44, 124.86, 124.14, 120.98, 112.55, 111.27, 95.90, 9.59.

### Example 19: 3-Methyl-5-((1S,2R)-2-phenylcyclopropane-1-carboxamido) benzofuran-2-carboxylic acid (GPR132-A-4)

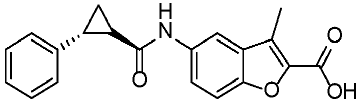

The preparation method was the same as that of Example 16, except that ethyl (*E*)-5-(3-(3-iodophenyl)acrylamido)-3-methylbenzofuran-2-carboxylate was used instead of ethyl 3-methyl-5-(3-phenylpropylamino)benzofuran-2-carboxylate to obtain the title compound. 121 mg of white solid was obtained, with a yield of 87.0%. ¹H NMR (400 MHz, DMSO-*d₆*) δ 10.40 (s, 1H), 8.14 (d, *J =* 2.4 Hz, 1H), 7.60 - 7.50 (m, 2H), 7.45 - 7.40 (m, 2H), 7.34 - 7.26 (m, 2H), 7.24 - 7.1 5 (m, 3H), 2.49 (s, 3H), 2.43 - 2.35 (m, 1H), 2.12 - 2.05 (m, 1H), 1.55 - 1.48 (m, 1H), 1.42 - 1.35 (m, 1H). ¹³C NMR (101 MHz, DMSO-*d₆*) δ 170.30, 161.53, 150.27, 142.38, 141.26, 135.57, 129.19, 128.83, 126.58, 126.37, 124.80, 120.77, 112.44, 111.00, 27.18, 25.35, 16.05, 9.55. HRMS (ESI) [M-H]⁻ Calculated value for C₂₀H₁₆NO₄: 334.1085; Measured value: 334.1083.

### Example 20: 3-Methyl-5-(4-oxo-4-phenylbutylamino)benzofuran-2-carboxylic acid (GPR132-A-5)

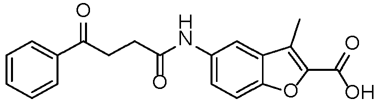

The preparation method was the same as Example 16, except that ethyl 3-methyl-5-(4-oxo-4-phenylbutylamino)benzofuran-2-carboxylate was used instead of ethyl 3-methyl-5-(3-phenylpropylamino)benzofuran-2-carboxylate to obtain the title compound. 38 mg of white solid was obtained, with a yield of 70.5%. ¹H NMR (400 MHz, DMSO-*d₆*) δ 13.39 (s, 1H), 10.20 (s, 1H), 8.12 (d, *J* = 2.0 Hz, 1H), 8.05 - 7.98 (m, 2H), 7.68 - 7.62 (m, 1H), 7.60 - 7.51 (m, 4H), 3.37 (t, *J =* 6.4 Hz, 2H), 2.76 (t, *J =* 6.4 Hz, 2H), 2.47 (s, 3H). ¹³C NMR (101 MHz, CDCl₃) δ 199.31, 170.78, 161.50, 150.20, 142.24, 137.01, 135.69, 133.66, 129.20, 129.12, 128.34, 124.88, 120.82, 112.41, 110.86, 33.58, 30.66, 9.55.

### Example 21: 3-Methyl-5-(5-phenylvaleramido)benzofuran-2-carboxylic acid (GPR132-A-6)

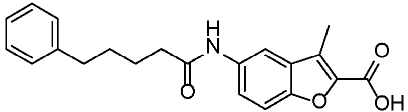

The preparation method was the same as that of Example 16, except that ethyl 3-methyl-5-(4-oxo-4-phenylbutylamino)benzofuran-2-carboxylate was used instead of ethyl 3-methyl-5-(3-phenylpropylamino)benzofuran-2-carboxylate to obtain the title compound. 199 mg of white solid was obtained, with a yield of 99%. ¹H NMR (400 MHz, DMSO-*d₆*) δ 13.36 (s, 1H), 10.01 (s, 1H), 8.12 (d, *J =* 2.0 Hz, 1H), 7.56 (d, *J =* 8.8 Hz, 1H), 7.53 (dd, *J =* 8.8, 2.0 Hz, 1H), 7.3 0 - 7.23 (m, 2H), 7.21 - 7.12 (m, 3H), 2.60 (t, *J =* 7.2 Hz, 2H), 2.49 (s, 3H), 2.35 (d, *J =* 7.2 Hz, 2H), 1.67 - 1.57 (m, 4H). ¹³C NMR (101 MHz, DM SO-d6) δ 171.58, 161.51, 150.26, 142.52, 142.28, 135.62, 129.12, 128.74, 128.70, 126.11, 124.84, 120.95, 112.33, 111.08, 36.68, 35.40, 31.11, 25.29, 9.56. HRMS (ESI) [M-H]⁻ Calculated value for C₂₁H₂₀NO₄: 350.1398; Measured value: 350.1390.

### Example 22: 3-Methyl-5-(3-phenylpropoxy)benzofuran-2-carboxylic acid (GPR132-A-7)

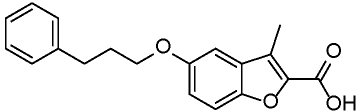

The preparation method was the same as that of Example 16, except that ethyl 3-methyl-5-(3-phenylpropyloxy)benzofuran-2-carboxylate was used instead of ethyl 3-methyl-5-(3-phenylpropylamino)benzofuran-2-carboxylate to obtain the title compound. 111 mg of white solid was obtained, with a yield of 87%. ¹HNMR (400 MHz, DMSO-*d₆*) δ 7.53 (d, *J =* 9.0 Hz, 1H), 7.34 - 7.15 (m, 6H), 7.11 (dd, *J =* 9.0, 2.6 Hz, 1H), 4.03 (t, *J =* 6.3 Hz, 2H), 2.81 - 2.73 (m , 2H), 2.50 (s, 3H), 2.10 - 2.01 (m, 2H). ¹³C NMR (101 MHz, DMSO-*d₆*) δ 161.1, 155.1, 148.5, 142.0, 141.4, 129.3, 128.4, 125.8, 124.4, 117.6, 112.6, 103.7, 67.4, 31.5, 30.4, 9.2. HRMS (ESI) [M-H]⁻ Calculated value for C₂₁H₂₃O₄: 339.1596; Measured value: 339.1592.

### Example 23: 3-Methyl-5-(N-benzyl-N-phenylethylsulfamoyl)benzofuran-2-carboxylic acid (GPR132-A-14)

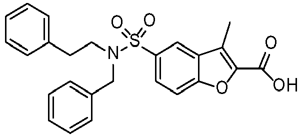

The preparation method was the same as that of Example 16, except that ethyl 3-methyl-5-(*N*-benzyl-*N*-phenylethylsulfamoyl)benzofuran-2-carboxylate was used instead of ethyl 3-methyl-5-(3-phenylpropylamino)benzofuran-2-carboxylate to obtain the title compound. A white solid of 75 mg was obtained with a yield of 84%. ¹H NMR (400 MHz, DMSO-*d₆*) δ 8.28 (s, 1H), 7.95 (d, *J* = 8.7 Hz, 1H), 7.84 (d, *J =* 8.8 Hz, 1H), 7.33 (dd, *J =* 16.2, 3.9 Hz, 5H), 7.24 - 7.05 (m, 3H) , 6.97 (d, *J= 7.1* Hz, 2H), 4.43 (s, 2H), 3.41 - 3.20 (m, 2H), 2.58 (s, 3H), 2.56 - 2.51 (m, 2H). ¹³C NMR (101 MHz, DMSO-*d₆*) δ 161.3, δ 155.5, 1 44.2, 138.7, 137.3, 135.3, 129.7, 129.0, 128.9, 128.8, 128.8, 128.1, 126.7, 126.5, 124.8, 122.0, 113.5, 51.8, 49.8, 34.8, 9.6. HRMS (ESI) [M-H]⁻ Calculated value for C₂₅H₂₄NO₅S: 450.1375; Measured value: 450.1378.

### Example 24: 3-Methyl-5-(N-(4-bromobenzyl)-N-phenylethylsulfamoyl)benzofuran- 2-carboxylic acid (GPR132-A-9)

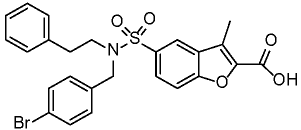

The preparation method was the same as Example 16, except that ethyl 3-methyl-5-(N-(4-bromobenzyl)-*N*-phenylethylsulfamoyl)benzofuran-2-carboxylate was used instead of ethyl 3-methyl-5-(3-phenylpropylamino)benzofuran-2-carboxylate to obtain the title compound. 227 mg of white solid was obtained, with a yield of 85%. ¹H NMR (400 MHz, DMSO-*d₆*) δ 8.15 (s, 1H), 7.93 - 7.69 (m, 2H), 7.42 (dd, *J =* 89.8, 8.2 Hz, 4H), 7.23 - 7.10 (m, 3H), 7.00 (d, *J =* 7.2 Hz, 2H), 4.39 (s, 2H), 3.39 - 3.27 (m, 2H), 2.56 (s, 5H). ¹³C NMR (101 MHz, DMSO-*d₆*) δ 162.0, 154.6, 148.2, 138.2, 136.7, 133.8, 131.3, 130.4, 130.0, 128.5, 128.3, 126.3, 124.7, 120.7, 120.7, 119.7, 112.6, 50.8, 49.6, 34.3, 9.1. HRMS (ESI) [M-H]⁻ Calculated value for C₂₅H₂₁BrNO₅S: 526.0329; Measured value: 506.0323.

### Example 25: 3-Methyl-5-(N-(4-(tert-butyloxycarbonyl)benzyl)-N-phenylethylsulfamoyl) benzofuran-2-carboxylic acid (GPR132-A-10)

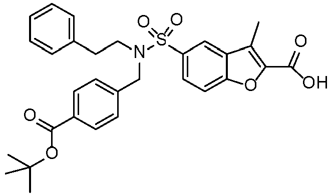

The preparation method was the same as Example 16, except that ethyl 3-methyl- 5-(*N-*(4-(tert-butyloxycarbonyl)benzyl)-*N*-phenylethylsulfamoyl)benzofuran-2-carboxylate was used instead of ethyl 3-methyl-5-(3-phenylpropylamino)benzofuran-2-carboxylate to obtain the title compound. 200 mg of white solid was obtained, with a yield of 77%. ¹H NMR (400 MHz, DMSO-*d*₆) δ 13.61 (s, 1H), 8.21 (s, 1H), 7.88 (dd, *J =* 47.6, 7.6 Hz, 4H), 7.42 (d, *J =* 8.1 Hz, 2H), 7.15 (dt, *J =* 16.9, 7.1 Hz, 3H), 7.01 (d, *J =* 6.2 Hz, 2H), 4.50 (s, 2H), 3.37 (s, 2H), 2.58 (d, *J =* 19.8 Hz, 5H), 1.52 (s, 9H). ¹³C NMR (101 MHz, DMSO) δ 165.1, 161.1, 155.5, 143.8, 142.5, 138.6, 135.2, 131.0, 129.9, 129.5, 129.0, 128.7, 128.6, 126.7, 126.6, 125.1, 122.0, 113.5, 81.1, 51.6, 50.3, 34.9, 28.2, 9.5.HRMS (ESI) [M-H]⁻ Calculated value for C₃₀H₃₀NO₇S: 548.1748; Measured value: 548.1738.

### Example 26: 3-Methyl-5-(N-(quinolin-8-ylmethyl)-N-phenylethylsulfamoyl)benzofuran-2-carboxylic acid (GPR132-A-11)

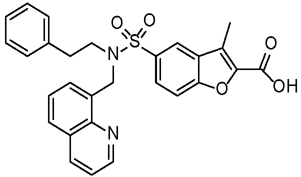

The preparation method was the same as Example 16, except that ethyl 3-methyl- 5-(*N-*(4-(tert-butyloxycarbonyl)benzyl)-*N*-phenylethylsulfamoyl)benzofuran-2-carboxylate was used instead of ethyl 3-methyl-5-(3-phenylpropylamino)benzofuran-2-carboxylate to obtain the title compound. 103 mg of white solid was obtained, with a yield of 99%. ¹H NMR (400 MHz, DMSO-*d*₆) δ 8.88 (s, 1H), 8.36 (d, *J =* 7.9 Hz, 1H), 8.20 (s, 1H), 7.91 (t, *J =* 9.1 Hz, 2H), 7.80 (d, *J =* 5.6 Hz, 2H), 7.68 - 7.48 (m, 2H), 7.12 (dd, *J =* 15.3, 6.9 Hz, 3H), 6.95 (d, *J =* 7.0 Hz, 2H), 5.10 (s, 2H), 3.60 - 3.38 (m, 2H), 2.73 - 2.60 (m, 2H), 2.54 (s, 3H). ¹³C NMR (101 MHz, DMSO-*d*₆) δ 161.1, 155.4, 150.2, 145.8, 143.6, 138.7, 137.2, 135.4, 135.0, 129.8, 129.4, 129.0, 128.7, 128.2, 126.8, 126.7, 125.2, 122.0, 121.9, 113.4, 50.8, 47.7, 35.1, 9.5. HRMS (ESI) [M-H]⁻ Calculated value for C₂₈H₂₃N₂O₅S: 499.1328; Measured value: 499.1321.

### Example 27: 3-Methyl-5-(N-phenethyl-N-phenylsulfamoyl)benzofuran-2-carboxylic acid (GPR132-A-12)

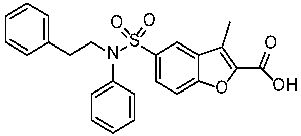

The preparation method was the same as Example 16, except that ethyl 3-methyl-5-(*N-*phenethyl-*N*-phenylsulfamoyl)benzofuran-2-carboxylate was used instead of ethyl 3-methyl-5-(3-phenylpropylamino)benzofuran-2-carboxylate to obtain the title compound. 50 mg of white solid was obtained, with a yield of 76%. ¹H NMR (400 MHz, DMSO-*d₆*) δ 7.73 (s, 1H), 7.67 (d, *J =* 8.7 Hz, 1H), 7.44 (d, *J =* 8.6 Hz, 1H), 7.35 (d, *J =* 6.8 Hz, 3H), 7.21 (dt, *J =* 27.5, 7.2 Hz, 3H), 7.12 (d, *J =* 7.1 Hz, 2H), 7.04 (d, *J =* 7.8 Hz, 2H), 3.82 (t, *J =* 7.2 Hz, 2H), 2.64 (t, *J =* 7.2 Hz, 2H), 2.45 (s, 3H). ¹³C NMR (101 MHz, DMSO-*d₆*) δ 154.9, 139.2, 138.7, 132.2, 130.6, 129.4, 129.2, 129.0, 128.7, 128.3, 126.8, 124.9, 121.0, 112.5, 51.5, 34.6, 9.4. HRMS (ESI) [M-H]⁻ Calculated value for C₂₄H₂₀NO₅S: 434.1062; Measured value: 434.1054.

### Example 28: 3-Methyl-5-(N,N-diphenylethylsulfamoyl)benzofuran-2-carboxylic acid (GPR132-A-15)

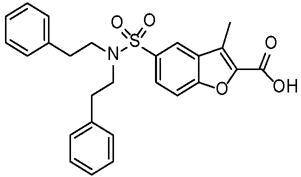

The preparation method was the same as Example 16, except that ethyl 3-methyl-5-(N,N-diphenylethylsulfamoyl)benzofuran-2-carboxylate was used instead of ethyl 3-methyl-5-(3-phenylpropylamino)benzofuran-2-carboxylate to obtain the title compound. 54 mg of white solid was obtained, with a yield of 78%. ¹H NMR (400 MHz, DMSO-*d₆*) δ 13.75 (s, 1H), 8.25 (s, 1H), 7.96 - 7.86 (m, 1H), 7.82 (d, *J =* 8.8 Hz, 1H), 7.32 - 7.22 (m, 4H), 7.18 (d, *J =* 7.4 Hz, 6H), 3.49 - 3.29 (m, 4H), 2.86 - 2.66 (m, 4H), 2.58 (s, 3H). ¹³C NMR (101 MHz, DMSO-*d₆*) δ 161.2, 155.5, 143.8, 139.0, 135.3, 129.6, 129.2, 128.8, 126.8, 126.6, 125.2, 121.8, 113.5, 49.8, 35.2, 9.6. HRMS (ESI) [M-H]⁻ Calculated value for C₂₆H₂₄NO₅S: 462.1375; Measured value: 462.1377.

### Example 29: 3-Methyl-5-(N-(4-(4-(methylsulfonyl)piperazin-1-yl)phenyl)- N-phenylethylsulfamoyl)benzofuran-2-carboxylic acid (GPR132-A-13)

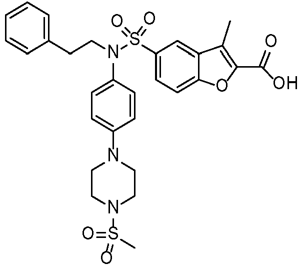

The preparation method was the same as that of Example 16, except that ethyl 3-methyl-5-(*N,N*-diphenylethylsulfamoyl)benzofuran-2-carboxylate was used instead of ethyl 3-methyl-5-(3-phenylpropylamino)benzofuran-2-carboxylate to obtain the title compound. 126 mg of white solid was obtained, with a yield of 99%. H NMR (400 MHz, DMSO-*d₆*) δ 7.75 (d, *J =* 1.9 Hz, 1H), 7.67 (d, *J =* 8.7 Hz, 1H), 7.46 (dd, *J* = 8.7, 1.9 Hz, 1H), 7.26 (dd, *J =* 8.0, 6.5 Hz, 2H), 7.22 - 7.10 (m, 3H), 6.96 - 6.82 (m, 4H), 3.75 (t, *J =* 7.3 Hz, 2H), 3.25 (q, *J =* 5.7 Hz, 8H), 2.93 (s, 3H), 2.63 (t, *J =* 7.3 Hz, 2H), 2.46 (s, 3H). ¹³C NMR (101 MHz, DMSO-*d₆*) δ 162.7, 154.9, 150.1, 138.8, 132.5, 130.5, 130.2, 129.8, 129.2, 128.7, 126.7, 125.0, 121.1, 118.3, 116.1, 112.5, 51.7, 48.0, 45.7, 34.6, 34.4, 9.5. HRMS (ESI) [M-H]⁻ Calculated value for C₂₉H₃₀N₃O₇S₂: 596.1525; Measured value: 596.1525.

### Example 30: 3-Methyl-5-(N-(4-phenoxyphenyl)-N-phenylethylsulfamoyl)benzofuran- 2-carboxylic acid (GPR132-A-16)

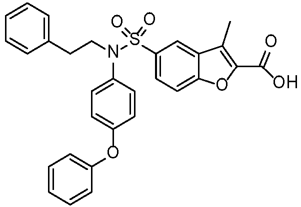

The preparation method was the same as Example 16, except that ethyl 3-methyl-5-(N,N-diphenylethylsulfamoyl)benzofuran-2-carboxylate was used instead of ethyl 3-methyl-5-(3-phenylpropylamino)benzofuran-2-carboxylate to obtain the title compound. 85 mg of white solid was obtained, with a yield of 81%. ¹H NMR (400 MHz, DMSO-*d₆*) δ 7.85 (d, *J=* 1.9 Hz, 1H), 7.75 (d, *J =* 8.8 Hz, 1H), 7.54 (dd, *J =* 8.7, 1.9 Hz, 1H), 7.46 - 7.38 (m, 2H), 7.25 (t, *J =* 7. 1 Hz, 2H), 7.22 - 7.11 (m, 4H), 7.07 - 6.99 (m, 4H), 6.97 - 6.91 (m, 2H), 3.81 (t, *J =* 7.2 Hz, 2H), 2.67 (t, *J* = 7.2 Hz, 2H), 2.51 (s, 3H). ¹³C NMR (101 MHz, DMSO-*d₆*) δ 162.0, 156.7, 156.6, 155.3, 138.7, 134.1, 132.6, 130.8, 130.6, 130.0, 129.2, 128.7, 126.7, 126.0, 124.3, 121.7, 119.4, 119.1, 112.9, 51.6, 34.6, 9.4. HRMS (ESI) [M-H]⁻ Calculated value for C₃₀H₂₄NO₆S: 526.1324; Measured value: 526.1316.

### Compound list

| No. | Chemical Name | Structure of Compound |
|---|---|---|
| GPR132-A-1 | 3-Methyl-5-(3-phenylpropylamino)benzofuran-2-carboxylic acid | 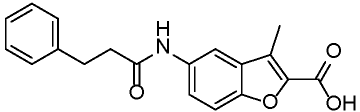 |
| GPR132-A-2 | 5-Cinnamylamino-3-methylbenzofuran-2-carboxylic acid | 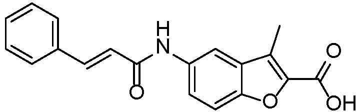 |
| GPR132-A-3 | (*E*)-5-(3-(3-iodophenyl)acrylamide)-3-methylbenzofuran-2-carboxylic acid | 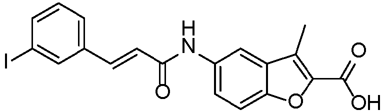 |
| GPR132-A-4 | 3-Methyl-5-((1*S*,2*R*)-2-phenylcyclopropyl-1-carboxamido)benzofuran-2-carboxylic acid | 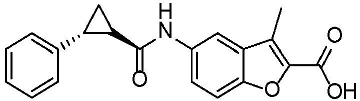 |
| GPR132-A-5 | 3-Methyl-5-(4-oxo-4-phenylbutylamino)benzofuran-2-carboxylic acid | 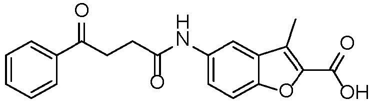 |
| GPR132-A-6 | 3-Methyl-5-(5-phenylvaleramido)benzofuran-2-carboxylic acid | 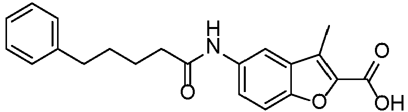 |
| GPR132-A-7 | 3-Methyl-5-(3-phenylpropoxy)benzofuran-2-carboxylic acid | 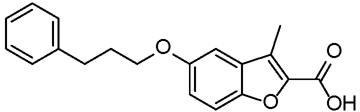 |
| B101 | 3-Methyl-5-(3-(m-tolyl)propoxy)benzofuran-2-carboxylic acid | 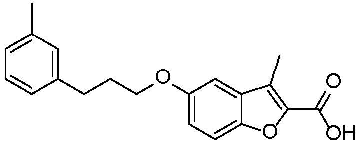 |
| B102 | 5-(3-(3-Methoxyphenyl)propoxy)-3-methylbenzofuran-2-carboxylic acid | 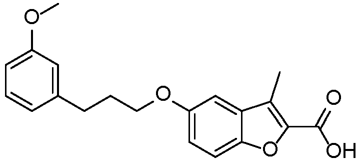 |
| B103 | 5-(3-(3-Chlorophenyl)propoxy)-3-methylbenzofuran-2-carboxylic acid | 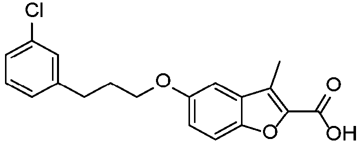 |
| GPR132-A-8 | 5-(3-(3-Methoxyphenyl)propylamino)-3-methylbenzofuran-2-carboxylic acid | 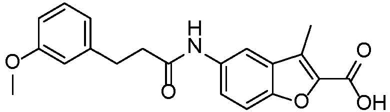 |
| B104 | 5-(3-(3-Chlorophenyl)propylamino)-3-methylbenzofuran-2-carboxylic acid | 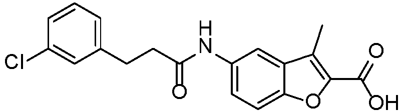 |
| B105 | 3-Methyl-5-(3-(m-tolyl)propylamino)benzofuran-2-carboxylic acid | 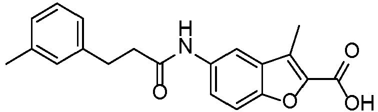 |
| B106 | 5-(3-(4-(Chloromethyl)phenyl)propylamin o)-3-methylbenzofuran-2-carboxylic acid | 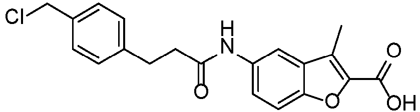 |
| B107 | 5-(3-(Benzo[*d*][1,3]dioxin-4-yl)propylamino)-3-methylbenzofuran-2-carboxylic acid | 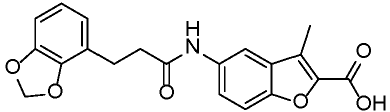 |
| B108 | 5-(3-(3-Ethynylphenyl)propylamino)-3-methylbenzofuran-2-carboxylic acid | 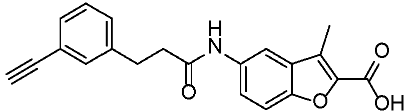 |
| B109 | 3-Methyl-5-(3-(methylamino)phenyl)propylamin o)benzofuran-2-carboxylic acid | 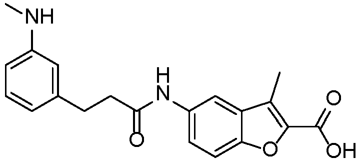 |
| B110 | 5-(3-(3-Cyanophenyl)propionamido)-3-methylbenzofuran-2-carboxylic acid | 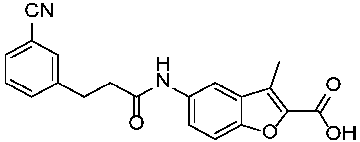 |
| B111 | 3-Methyl-5-(3-(4-(trifluoromethyl)phenyl)propylami no)benzofuran-2-carboxylic acid | 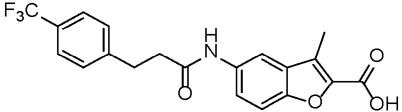 |
| B112 | 3-Methyl-5-(3-(pyridin-4-yl)propylamino)benzofuran-2-carboxylic acid | 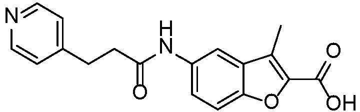 |
| B113 | 5-(3-(4-Methoxypyridin-2-yl)propylamino)-3-methylbenzofuran-2-carboxylic acid | 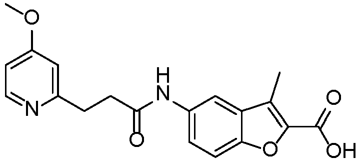 |
| B114 | (*E*)-5-(3-(5-Chloropyridin-2-yl)acrylamido)-3-methylbenzofuran-2-carboxylic acid | 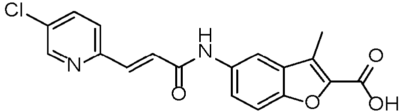 |
| B115 | 5-(3-(1*H*-Pyrrol-2-yl)propylamino)-3-methylbenzofuran-2-carboxylic acid | 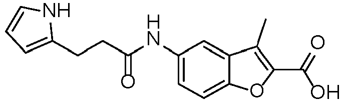 |
| B116 | 3-Methyl-5-(3-(thiophen-2-yl)propionamido)benzofuran-2-carboxylic acid | 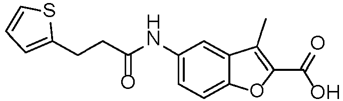 |
| B117 | 5-(3-(5-Chlorothiophen-2-yl)propylamino)-3-methylbenzofuran-2-carboxylic acid | 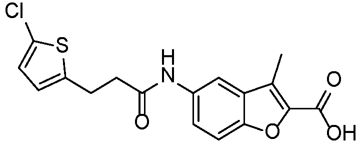 |
| B118 | 3-Methyl-5-(3-(5-methylthiophen-2-yl)propylamino)benzofuran-2-carboxylic acid | 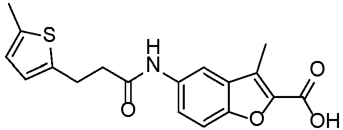 |
| B119 | 3-Methyl-5-(pent-4-eneylamino)benzofuran-2-carboxylic acid | 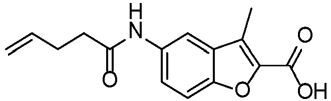 |
| B120 | 3-Methyl-5-(pent-4-ynylamino)benzofuran-2-carboxylic acid | 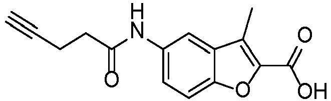 |
| B121 | 3-Methyl-5-(3-(naphthalen-1-yl)propylamino)benzofuran-2-carboxylic acid | 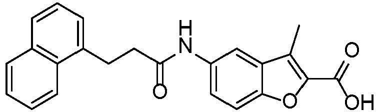 |
| B 122 | 5-(3-([1,1'-Biphenyl]-3-yl)propylamino)-3-methylbenzofuran-2-carboxylic acid | 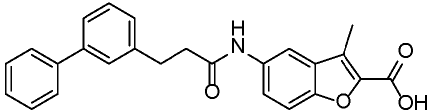 |
| B123 | 5-(3-(1H-indol-3-yl)propylamino)-3-methylbenzofuran-2-carboxylic acid | 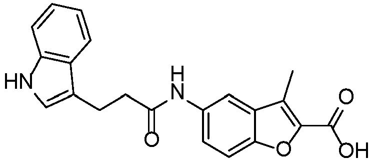 |
| B124 | 5-(4-(4-Fluorophenyl)-4-oxobutylamino)-3-methylbenzofuran-2-carboxylic acid | 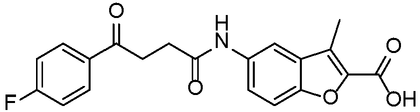 |
| B125 | 5-(4-(4-Chlorophenyl)-4-oxobutylamino)-3-methylbenzofuran-2-carboxylic acid | 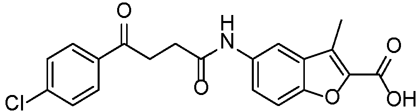 |
| B126 | N-Hydroxy-3-methyl-5-(3-phenylpropylamino)benzofuran-2-carboxamide | 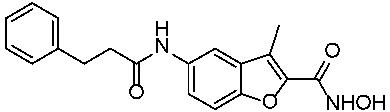 |
| B127 | 3-Chloro-5-(3-phenylpropylamino)benzo[b]thiop hene-2-carboxylic acid | 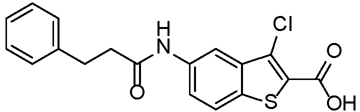 |
| B128 | 3-Methyl-5-(3-phenylpropylamino)benzo[b]thiop hene-2-carboxylic acid | 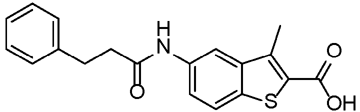 |
| B129 | 3-Chloro-5-(3-phenylpropylamino)-1H-indole-2-carboxylic acid | 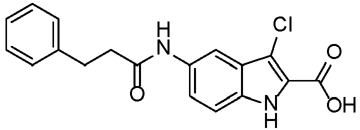 |
| B130 | 3-Methyl-5-(3-phenylpropylamino)-1H-indole-2-carboxylic acid | 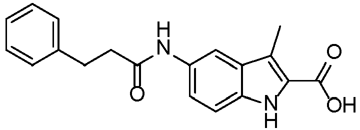 |
| B131 | 3-(2-Fluoro-5-(3-phenylpropylamino)phenyl)propio nic acid | 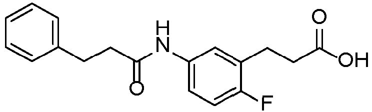 |
| B132 | 3-(2-Fluoro-5-(3-phenylpropylamino)phenyl)butyric acid | 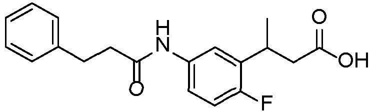 |
| B133 | 3-(2-Fluoro-5-(3-phenylpropyloxy)phenyl)propionic acid | 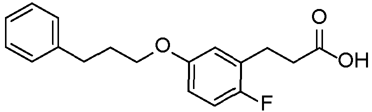 |
| GPR132-A-9 | 3-Methyl-5-(N-(4-bromobenzyl)-*N-*phenylethylsulfamoyl)benzofuran-2-carboxylic acid | 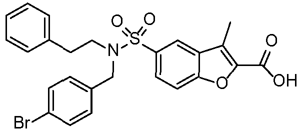 |
| GPR132-A-10 | 3-Methyl-5-(N-(4-(tert-butyloxycarbonyl)benzyl)-N-phenylethylsulfamoyl)benzofuran-2-carboxylic acid | 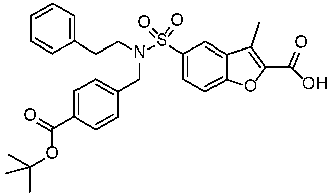 |
| GPR132-A-11 | 3-Methyl-5-(N-(quinolin-8-ylmethyl)-N-phenylethylsulfamoyl)benzofuran-2-carboxylic acid | 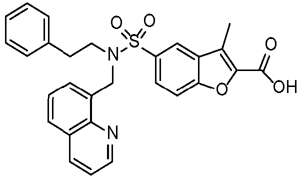 |
| GPR132-A-12 | 3-Methyl-5-(N-phenylethyl-N-phenylsulfamoyl)benzofuran-2-carboxylic acid | 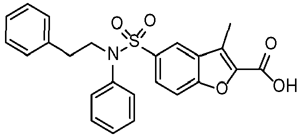 |
| GPR132-A-13 | 3-Methyl-5-(N-(4-(4-(methylsulfonyl)piperazin-1-yl)phenyl)-N-phenylethylsulfamoyl)benzofuran-2-carboxylic acid | 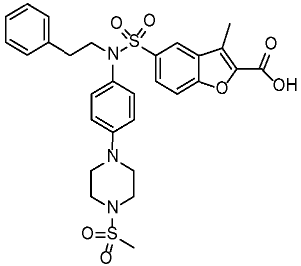 |
| GPR132-A-14 | 3-Methyl-5-(N-benzyl-N-phenylethylsulfamoyl)benzofuran-2-carboxylic acid | 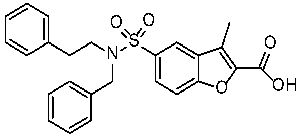 |
| GPR132-A-15 | Example 28 3-Methyl-5-(N,N-diphenylethylsulfamoyl)benzofura n-2-carboxylic acid | 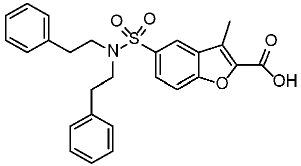 |
| GPR132-A-16 | 3-Methyl-5-(N-(4-phenoxyphenyl)-N-phenylethylsulfamoyl)benzofuran-2-carboxylic acid | 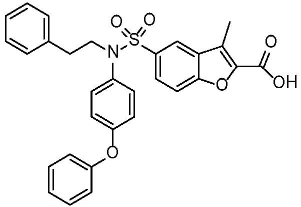 |

The above compounds could be prepared by referring to the above method.

### Test of biological activity

### 1.1 G protein activation

Gi BRET probes were generated according to previous literature (Olsen et al., 2020), and included Gαi-RLuc8, Gβ3, and Gγ9-GFP2. GPR132 and Gi BRET probes were transiently co-transfected in HEK293 cells. 24 hours after transfection, the cells were distributed into 96-well microplates at a density of 5×10⁴ cells per well and cultured for another 24 hours. The cells were washed twice with Tyrode buffer and stimulated with compound ligands at different concentrations (10⁻¹² to 10⁻⁵ M) for 2 minutes. After adding coelenterazine, the substrate of luciferase, 400a (5 µM), BRET signal was measured using a Mithras LB940 microplate reader equipped with a BRET filter set. The BRET signal was calculated as the emission light ratio at 510 nm/400 nm.

The compounds with lower EC₅₀ values in the above experiments had good GPR132 agonist activity.

### 1.2 Cell culture and subculture

Human embryonic kidney cell line HEK293A was sub-cultured in MEM medium containing penicillin (final concentration was 100 U/mL), streptomycin (final concentration was 100 µg/mL) and 10% FBS, and underwent constant temperature incubation at 37 °C in a humidified atmosphere comprising 95% air and 5% carbon dioxide. When the cells were 90% confluent, the old medium was discarded, and the cells were washed twice with 2 mL of PBS. After discarding PBS, 2 mL of 0.25% trypsin-0.25% EDTA mixed digestion solution was added, and the cells were observed with a microscope. When the cells became round, 2 mL of complete medium was quickly added to terminate digestion, and the cells were gently pipetted and collected, centrifuged at 800 rpm and 4 °C for 5 min; the supernatant was discarded; the cells were resuspended with complete medium, cultured in bottles, and the medium was changed every other day.

Human liver cancer cell line HepG2 was sub-cultured in DMEM medium containing penicillin (final concentration was 100 U/mL), streptomycin (final concentration was 100 µg/mL) and 10% FBS, and the rest operations were the same as the HEK293A sub-culture.

### 1.3 Experiment of inhibiting cell proliferation

The inhibition ability of the compounds on cell proliferation was determined by MTT method. HepG2 and HEK293 cells were inoculated in 96-well plates at a density of 30%. After incubation for 18 h to allow the cells to adhere to wall, the original culture medium was removed, and the compound to be tested that had a given concentration was added to each well. The control group was added with an equal amount of DMSO. Three replicate wells were set for each compound. After 48 h of drug administration, the cell viability was determined by MTT method. The blank group was the well without cells. Then the OD value was read at 490 nm using a microplate reader. The calculation formula for cell viability was Cell viability (%) = (OD _{to be tested} - OD _{blank}) / (OD _{control} - OD _{blank}) × 100.

In the above experiment, the compounds with high viability and high CC50 values for HEK293 cells had good safety. The compounds with high inhibition rate and low EC₅₀ values for HepG2 cells could better inhibit the proliferation of tumor cells.

### 1.4 Determination of level of tumor necrosis factor (TNF-α) as cell inflammatory marker

HepG2 cells were inoculated in 96-well plates at a density of 30%. After incubation for 18 h to allow the cells to adhere to wall, the original culture medium was removed, and each well of the drug group was added with LPS culture medium (LPS concentration was 1 µg/mL), the control group was added with an equal amount of DMSO, and incubation was performed overnight. Subsequently, the original culture medium was removed, and the compound to be tested that had a given concentration was added, and the control group was added with an equal amount of DMSO. Three replicate wells were set for each compound. After 48 h of drug administration, the cell supernatant was collected with a sterile tube, and centrifuged for about 20 minutes (2000 to 3000 rpm). The supernatant was carefully collected. The TNF-α level was then determined using ELISA method. The specific steps were as follows:
▪ Standard sample addition: Standard sample wells and sample wells were set, and 50 µL of standard sample at different concentrations was added to each standard sample well.
▪ Sample addition: Blank wells (blank control wells was not added with sample and ELISA reagent, and the rest operations were the same) and test sample wells were set. First, 40 µL of sample diluent was added to each test sample well on an ELISA plate, and then 10 µL of the sample to be tested (the final dilution ratio of sample was 5) was added. The sample was added to the bottom of the well of the ELISA plate, not touching the well wall as much as possible, and then the gentle shaking was carried out for mixing.
▪ Enzyme addition: 100 µL of ELISA reagent was added to each well, except for the blank wells.
▪ Incubation: The plate was sealed with a sealing film and incubated at 37°C for 60 minutes.
▪ Solution preparation: 20-Fold concentrated washing solution was diluted by 20-fold with distilled water for later use.
▪ Washing: The sealing film was carefully peeled off, the liquid was discarded, the plate was spin-dried; each well was filled with the washing solution, allowed to stand for 30 seconds and then the washing solution was discarded. This operation was repeated 5 times, and pat-drying was performed.
▪ Color development: First, 50 µL of color developer A was added to each well, then 50 µL of color developer B was added, gently shaken and mixed, and color development was performed at 37°C in the dark for 15 minutes.
▪ Termination: 50 µL of termination liquid was added to each well to stop the reaction (the blue color immediately turned yellow).
▪ Determination: Zero was set with the blank well, and the absorbance (OD value) at 450 nm of each well was measured in sequence. The determination should be performed within 15 minutes after adding the termination liquid.

TNF-α content in the sample to be tested could be calculated by drawing a standard curve based on the standard samples. TNF-α reduction rate was further calculated. The formula for calculating TNF-α reduction was Reduction rate (%) = TNF-α content _{control} - TNF-α content to be _{tested}/TNF-α content _{control} × 100.

In the above experiment, the compounds with higher TNF-α reduction rate and lower EC₅₀ value could better reduce the level of inflammation.

### 1.5 Determination of cell TC/TG

Cell TC/TG was determined using Nanjing Jiancheng TG test kit and TC test kit. HepG2 cells were incubated with 0.5 mmol/L FFA (oleic acid and palmitate mixed in a ratio of 2:1) and solvent or compounds of different concentrations (20 µM, 40 µM, 80 µM) for 24 h. The culture medium was discarded, and the cells were washed once with 0.01 M, pH 7.4 PBS. The cells were scraped with a cell scraper, added with 2 to 5 mL of 0.01 M, pH 7.4 PBS; the cell suspension was collected, centrifuged at 1000 × g and 4 °C for 10 min to collect the cells. Isopropanol was added according to the ratio of 300 to 500 µL of homogenization medium per 10⁶ cells, mechanical homogenization was performed, and the cells were fully broken until there was no obvious cell precipitation. Centrifugation was performed at 10000 × g and 4 °C for 10 min; the supernatant was taken and placed on ice for later test. 2.5 µL of distilled water; standard or sample was taken and mixed with 250 µL of distilled water respectively, incubated at 37 °C for 10 minutes, and the absorbance of each well was measured at 510 nm with a microplate reader. The calculation formula of TC/TG concentration was sample concentration (mmol/g protein) = (OD ₛₐₘₚₗₑ - OD _{blank}) / (OD _{standard} - OD _{blank}) × standard concentration (mmol/L) / protein concentration (g protein /L).

### Protein quantification by BCA method

Preparation of protein standards: a. 1.2 mL of protein standard preparation solution was added to a tube of protein standard (30 mg BSA) and fully dissolved, and a 25 mg/mL protein standard solution was prepared. b. an appropriate amount of a 25 mg/mL protein standard was diluted to a final concentration of 0.5 mg/mL. Preparation of BCA working solution: According to the number of samples, an appropriate amount of BCA working solution was prepared by thoroughly mixing 50 volumes of BCA reagent A with 1 volume of BCA reagent B (50:1). Determination of protein concentration: a. 0, 1, 2, 4, 8, 12, 16, and 20 µL of the standard was added to the standard wells of a 96-well plate, respectively, and standard diluent was added to make up to 20 µL, resulted with standard samples with concentrations of 0, 0.025, 0.05, 0.1, 0.2, 0.3, 0.4, and 0.5 mg/mL, respectively. b. an appropriate volume of sample was added to sample wells of the 96-well plate. If the sample was less than 20 µL, the standard diluent was added to make up to 20 µL. c. 200 µL of BCA working solution was added to each well and placed at 37°C for 20 to 30 minutes. d. the absorbance at wavelength of A562 nm was measured by a microplate reader. e. the protein concentration of the sample was calculated based on the standard curve and the sample volume used.

In the above experiment, the compounds with higher TC/TG reduction rates and lower EC₅₀ values could better reduce the fat content in blood and/or liver.

### 1.6 Experiment on animals with acute hyperlipidemia

Male C57BL/6 mice were randomly divided into 8 groups, including Control group, Model group, SIPI-7623 groups (10 mg/kg, 30 mg/kg and 100 mg/kg) and test groups (3 doses). The Model group, SIPI-7623 group and test groups were intraperitoneally injected with 600 mg/kg Triton WR-1339, and the Control group was injected with the same volume of saline. The test compound was administered by gavage 1 hour before and 23 hours after the injection of the inducer; one hour later, blood and tissue samples were collected.

### 1.7 Experiment on animals with high-fat diet

Male C57BL/6 mice were randomly divided into 6 groups, including Control group, HFD group, SIPI-7623 group (40 mg/kg) and test groups (3 doses). The Control group was given a normal diet, and the HFD group, SIPI-7623 group and test groups were given a high-fat diet. The Control group and the HFD group were given solvent by gavage, and the other groups were given corresponding doses of the test compound for treatment. The weight of mice was recorded every day. Blood and tissue samples were collected after 8 weeks of the gavage of the test compound. The composition of the high-fat diet was shown in the table below.

Compositions of standard chow and high fat diet for mice

| Ingredient | Standard chow (g/kg) | Kcal/kg | High fat diet (g/kg) | Kcal/kg |
|---|---|---|---|---|
| Corn starch | 397.5 | 1590 | 115.5 | 462 |
| Casein | 200 | 800 | 200 | 800 |
| Sucrose | 100 | 400 | 100 | 400 |
| Dextrin starch | 132 | 528 | 132 | 528 |
| Lard | - | - | 312 | 2808 |
| Soybean oil | 70 | 630 | 40 | 360 |
| Cellulose | 50 | - | 50 | - |
| Minerals | 35 | - | 35 | - |
| Vitamins | 10 | - | 10 | - |
| L-cysteine | 3 | - | 3 | - |
| Choline | 2.5 | - | 2.5 | - |
| Total | 1000 | 3948 | 1000 | 5358 |

### 1.8 Experiment on animals with diet-induced obesity

Male C57BL/6 mice were divided into 2 groups: Control group that had a normal diet, and HFD group that was given a high-fat diet. After 12 weeks of high-fat diet, the HFD group was randomly divided into 9 groups: HFD group, test substance groups (3 doses), SIPI-7623 group (40 mg/kg), Atorvastatin group (10 mg/kg), and group of test substance (10 mg/kg) + Atorvastatin (10 mg/kg). The Control group and HFD group were given solvent by gavage, and the other groups were treated with corresponding doses of compounds. Body weight of mice was recorded weekly. After 8 weeks of gavage treatment with compounds, blood samples and tissue samples were collected. The composition of the high-fat diet is shown in the table above.

### 1.9 Animal experiment on db/db mice

Male db/db mice were randomly divided into 5 groups, and the experimental groups were db/db group, test substance group (3 doses), and pioglitazone (7 mg/kg). The db/db group was treated with solvent by gavage, and the other groups were treated with corresponding compounds by gavage for 4 weeks. Weight of the mice was recorded weekly, and fasting blood glucose was tested. After 4 weeks of the treatment with compounds by gavage, blood samples and tissue samples were collected for subsequent experiments.

### 2.0 Determination of mouse blood glucose

1. Roche blood glucose meter, containing blood glucose meter, blood collection pen, and blood glucose test strips.
2. The tip of mouse tail was wiped with an alcohol cotton ball, and sampling needle on the blood collection pen was opened to pierce the tip of the mouse tail; the tail tip blood was dipped with a blood glucose test strip, and the blood must completely cover the test area of the test strip; the blood glucose meter was opened, the blood glucose test strip was inserted into the blood glucose meter, and the blood glucose value was read and recorded. The sampling needle and blood glucose test strip should be replaced before each mouse was tested for blood glucose.

### 2.1 Glucose tolerance test (GTT)

The glucose tolerance test was performed in the 5^{th} week after the administration of the test substance by gavage (the 17^{th} week of the experiment). After fasting for 16 h, the mice were intraperitoneally injected with glucose (1.5 g/kg), and the blood glucose levels of mice were measured with a blood glucose meter at 0, 15, 30, 60, 90, and 120 min after glucose injection, respectively.

### 2.2 Insulin tolerance test (ITT)

The insulin tolerance test was performed in the 6^{th} week after the administration of the test substance by gavage (the 18^{th} week of the experiment). After fasting for 4 h, the mice were intraperitoneally injected with insulin (1 U/kg), and the blood glucose levels of mice were measured with a blood glucose meter at 0, 15, 30, 60, 90, and 120 min after insulin injection, respectively.

### 2.3 Animal sampling

1. After the mice were weighed, they were anesthetized by intraperitoneal injection of sodium pentobarbital.
2. Blood was collected from the inner canthus, 20 µL of whole blood was taken, diluted with whole blood diluent, and blood routine tests were performed; the remaining whole blood was left to stand at room temperature for 2 h, centrifuged at 3000 rpm and 4 °C for 10 min, and the upper serum was taken for blood biochemical tests.
3. After the blood sampling of the mice was completed, the chest and abdominal cavities were opened, and the liver, intestine, white fat and brown fat tissues were taken out, respectively.
4. A portion of the liver was fixed with 4% formaldehyde for paraffin sectioning and frozen sectioning, and the remaining tissue was frozen at -80 °C.
5. After the intestinal tissue was rinsed with PBS, intestinal epithelial cells were scraped and frozen at -80 °C.
6. The white fat and brown fat tissues were weighed.

### 2.4 Detection of cholesterol and triglyceride in liver

1. An appropriate amount of liver tissue was taken, added with grinding beads and subjected to tissue homogenization using PBS as buffer.
2. The homogenate was divided into two equal parts, chloroform and methanol were added to one part for extraction, and concentrations of cholesterol and triglycerides were measured. Chloroform-methanol extract solution (chloroform: methanol = 2:1) at 4 times the volume was added to one of the tubes, vortexed to mix thoroughly, allowed to stand until stratification, and centrifuged at 2000 rpm and 4 °C for 30 min; the lower layer liquid was pipetted and placed into a new centrifuge tube, evaporated to remove the solvent in a vacuum concentrator, and then an appropriate amount of 3% Triton X-100 was added, and dissolution was carried out in a constant temperature water bath shaker at 50 to 60 °C. TC and TG kits were used to determine concentrations.
5. Another tube of homogenate was taken, centrifuged at 12000 rpm and 4 °C for 15 min, the supernatant was taken, and the BCA kit was used to determine the protein concentration.
6. Lipid content = lipid concentration/protein concentration

### 2.5 Staining liver with Oil Red O

1. Fresh liver tissues of the same size and location were taken, placed in 4% neutral formaldehyde for fixation, and send them to a testing company to prepare slices and perform Oil Red O staining.
2. The prepared slices were taken back, scanned with a scanner, and photographed.

### 2.6 HE staining of tissues

1. Fresh liver tissues of the same size and location were taken, placed in 4% neutral formaldehyde for fixation, and send them to a testing company to prepare slices and perform HE staining.
2. The prepared slices were taken back, scanned with a scanner and photographed.

### 2.7 Animal experiment on mice with inflammatory bowel disease

### Modeling with DSS

DSS is a polyanionic derivative of dextran, formed by the esterification reaction of dextran and chlorosulfonic acid, with a molecular formula of (C₆H₇Na₃O₁₄S₃)ₙ, MW: 36000~50000, and a sulfur content of 17% to 20%. DSS is often used to induce colitis models, although its induction mechanism has not yet been clarified. Current studies mainly believe that DSS increases intestinal permeability, destroys intestinal mucosal barrier, upregulates certain cytokines (tumor necrosis factor, interleukin, interferon, IL-10 and IL-12), activates certain pathways (NF-κB pathway and TRPV1 pathway), or is related to intestinal microbiota imbalance. According to the administration time and administration cycle, there are two types of colitis models: acute model and chronic model.

Male C57BL/6 mice were randomly divided into 6 groups, with a control group and experimental groups. The experimental groups were model group, test group (3 doses) and positive compound ,tofacitinib, group (10 mg/kg). The control group was treated with solvent by gavage, and the other groups were treated with corresponding compounds by gavage. The weight of mice was recorded every day. After the treatment with compounds by gavage, blood samples and tissue samples were collected for subsequent experiments.

### Chronic colitis modeling

The chronic colitis model could be established with low concentration DSS, but the administration time was longer. For example, it could be made by giving mice 1% to 3% DSS via free drinking for several weeks. The manifestations of a chronic colitis model included: obvious shortening of mouse colon, epithelial hyperplasia, mucosal fibrosis and lymphadenopathy; as well as granulation tissue hyperplasia and tumor-like changes in a small number of animals.

Male C57BL/6 mice were randomly divided into 6 groups, with a control group and experimental groups. The experimental groups include model group, test groups (3 doses) and positive compound tofacitinib group (10 mg/kg). The control group was given solvent by gavage, and the other groups were given corresponding compound by gavage. The weight of mice was recorded every day. After the treatment with compounds by gavage, blood samples and tissue samples were collected for subsequent experiments.

### Acute colitis modeling

Acute colitis modeling is also one of the commonly used colitis models. Because of its simple preparation, high success rate, and similarity to human UC lesions, it is an ideal model for studying the pathogenesis of UC and evaluating drug efficacy. Acute modeling is generally established with a higher concentration of DSS and a shorter administration time. For example, it could be made by giving hamsters 3% to 5% DSS via free drinking for one week. The manifestations of an acute colitis model included: congestion, edema, shortening, brittleness, and increased weight-to-length ratio in colon, varying degrees of colon ulcers, mucosal edema, goblet cell loss, crypt swelling and destruction, varying degrees of inflammatory cell infiltration in mucosa and submucosal layer, and epithelial cell damage.

Male C57BL/6 mice were randomly divided into 6 groups, with a control group and experimental groups. The experimental groups included model group, test groups (3 doses) and positive compound tofacitinib group (10 mg/kg). The control group was treated with solvent by gavage, and the other groups were treated with corresponding compounds by gavage. Weight of mice was recorded every day. After the treatment with compounds by gavage, blood samples and tissue samples were collected for subsequent experiments.

### Experimental results

GPR132 agonist activity results indicated:
A: 0.01 µM < EC₅₀ < 1 µM
B: 1 µM < EC₅₀ < 10 µM
C: 10 µM < EC₅₀ < 100 µM

**Table 1: GPR132 agonist activity of some compounds**

| Test compound | Compound No. | GPR132 EC₅₀ (µM) |
|---|---|---|
| Example 16 | GPR132-A-1 | 6.5 |
| Example 18 | GPR132-A-3 | 0.51 |
| Example 23 | GPR132-A-14 | 3.8 |
| Example 24 | GPR132-A-9 | 0.30 |
| Example 28 | GPR132-A-15 | 2.1 |

**Table 2: GPR132 agonist activity of some compounds**

| Test compound | Compound No. | GPR132 EC₅₀ (µM) |
|---|---|---|
| Example 17 | GPR132-A-2 | A |
| Example 19 | GPR132-A-4 | B |
| Example 20 | GPR132-A-5 | A |
| Example 21 | GPR132-A-6 | B |
| Example 22 | GPR132-A-7 | A |
| Example 25 | GPR132-A-10 | A |
| Example 26 | GPR132-A-11 | B |
| Example 27 | GPR132-A-12 | B |
| Example 29 | GPR132-A-13 | B |
| Example 30 | GPR132-A-16 | A |

**Table 3: Activity of some compounds in reducing TNF-α**

| Test compound | Compound No. | EC₅₀ (µM) |
|---|---|---|
| Example 16 | GPR132-A-1 | 10.2 |
| Example 18 | GPR132-A-3 | 1.0 |
| Example 23 | GPR132-A-14 | 5.9 |
| Example 24 | GPR132-A-9 | 0.7 |
| Example 28 | GPR132-A-15 | 3.1 |

**Table 4: Activity of some compounds in reducing TNF-α**

| Activity results in reducing TNF-α indicated: | | |
|---|---|---|
| Test compound | Compound No. | EC₅₀ (µM) |
| Example 17 | GPR132-A-2 | A |
| Example 19 | GPR132-A-4 | B |
| Example 20 | GPR132-A-5 | A |
| Example 21 | GPR132-A-6 | A |
| Example 22 | GPR132-A-7 | A |
| Example 25 | GPR132-A-10 | A |
| Example 26 | GPR132-A-11 | B |
| Example 27 | GPR132-A-12 | B |
| Example 29 | GPR132-A-13 | B |
| Example 30 | GPR132-A-16 | B |

| | | |
|---|---|---|
| A: 0.01 µM < EC₅₀ < 10 µM B: 10 µM < EC₅₀ < 50 µM C: 50 µM < EC₅₀ < 100 µM | | |

The experimental results showed that the compounds of the present invention had one or more of the following effects:
(1) having good GPR132 agonist activity;
(2) having good safety;
(3) being able to improve glucose tolerance;
(4) being able to improve insulin resistance;
(5) being able to lower blood sugar levels;
(6) being able to lower blood lipid levels;
(7) being able to lower fat content in liver;
(8) being able to inhibit proliferation of tumor cells;
(9) being able to reduce inflammation levels; and
(10) being able to improve intestinal diseases.

In summary, the compounds of the present invention could be used as GPR132 agonists for the prevention and/or treatment of diseases related to GPR132, such as tumors, metabolic diseases or autoimmune diseases.

Although the specific embodiments of the present invention have been described in detail, those skilled in the art will understand that various modifications and substitutions can be made to those details based on all the teachings disclosed, and these changes are within the scope of protection of the present invention. The full scope of the present invention is given by the appended claims and any equivalents thereof.

## Claims

1. A compound of Formula I, or a stereoisomer, prodrug, crystal form, pharmaceutically acceptable salt, pharmaceutically acceptable solvate or pharmaceutically acceptable ester of the compound, 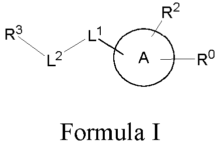 wherein:
Ring A is selected from the group consisting of benzofuran ring, benzothiophene ring, indole ring and benzene ring;
preferably, Ring A is selected from the group consisting of 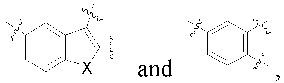 X is selected from the group consisting of O, S and NH, and X is preferably selected from O;
R⁰ is selected from the group consisting of 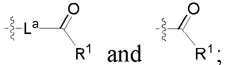
preferably, R⁰ is 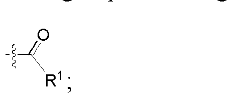
L^{a} is selected from the group consisting of C1-C6 alkylene;
preferably, L^{a} is selected from the group consisting of 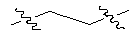 and 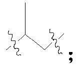
R¹ is selected from the group consisting of hydroxyl, C1-C6 alkoxy and -NH-OH;
R² is selected from the group consisting of hydrogen, halogen and C1-C6 alkyl;
R³ is selected from the group consisting of C2-C6 alkenyl, C2-C6 alkynyl, phenyl, naphthyl and 5- to 10-membered heteroaryl; optionally, the phenyl, naphthyl and 5- to 10-membered heteroaryl are each independently substituted by 1 to 5 groups selected from the group consisting of R^{x}, or the phenyl, naphthyl and 5- to 10-membered heteroaryl are each independently substituted by 2 adjacent groups, and the 2 adjacent groups and the carbon atoms to which they are respectively connected together form a 5- to 6-membered heterocyclic ring;
R^{x} is selected from the group consisting of C1-C6 alkyl, halogen, C1-C6 alkoxy, C1-C6 haloalkyl, C2-C6 alkynyl, cyano, phenyl and -NR^{m}Rⁿ;
R^{m} and Rⁿ are each independently selected from the group consisting of hydrogen and C1-C6 alkyl;
L¹ is selected from the group consisting of 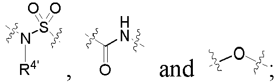
preferably, L¹ is selected from the group consisting of 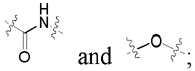
L² is selected from the group consisting of 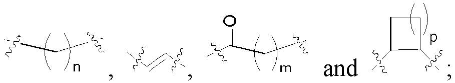 n is selected from the group consisting of 1, 2, 3, 4, 5 and 6;
m is selected from the group consisting of 1, 2, 3, 4, 5 and 6;
p is selected from the group consisting of 0, 1, 2 and 3;
R^{4'} is selected from the group consisting of phenyl-(CH₂)ᵣ- and 5- to 10-membered heteroaryl-(CH₂)ᵣ-, optionally, the phenyl and 5- to 10-membered heteroaryl are each independently substituted by 1 to 5 groups selected from the group consisting of R^{a};
r is selected from the group consisting of 0, 1, 2, 3, 4 and 5;
R^{a} is selected from the group consisting of halogen, C1-C6 alkyl-O-C(=O)-, and 5- to 6-membered saturated heterocyclyl optionally substituted by 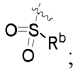
R^{b} is selected from the group consisting of hydrogen and C1-C6 alkyl.

2. The compound, or stereoisomer, prodrug, crystal form, pharmaceutically acceptable salt, pharmaceutically acceptable solvate or pharmaceutically acceptable ester of the compound, according to claim 1, wherein R¹ is selected from the group consisting of hydroxyl and C1-C6 alkoxy;
preferably, R¹ is selected from the group consisting of hydroxyl and ethoxy;
more preferably, R¹ is hydroxyl;
or preferably, R¹ is selected from the group consisting of hydroxyl, ethoxy and -NH-OH.

3. The compound, or stereoisomer, prodrug, crystal form, pharmaceutically acceptable salt, pharmaceutically acceptable solvate or pharmaceutically acceptable ester of the compound, according to any one of claims 1 to 2, wherein R² is selected from the group consisting of halogen and C1-C6 alkyl;
preferably, R² is selected from the group consisting of fluorine, chlorine and methyl;
or preferably, R² is selected from the group consisting of C1-C6 alkyl;
or more preferably, R² is methyl.

4. The compound, or stereoisomer, prodrug, crystal form, pharmaceutically acceptable salt, pharmaceutically acceptable solvate or pharmaceutically acceptable ester of the compound, according to any one of claims 1 to 3, wherein R³ is selected from the group consisting of C2-C6 alkenyl, C2-C6 alkynyl, phenyl, naphthyl, pyridyl, pyrrolyl, thienyl and indolyl; optionally, the phenyl, naphthyl, pyridyl, pyrrolyl, thienyl and indolyl are each independently substituted by 1 to 2 (preferably 1) groups selected from the group consisting of R^{x}, or the phenyl, naphthyl, pyridyl, pyrrolyl, thienyl and indolyl are each independently substituted by 2 adjacent groups, and the 2 adjacent groups and the carbon atoms to which they are respectively connected together form a 5-to 6-membered heterocyclic ring;
preferably, R³ is selected from the group consisting of C2-C6 alkenyl, C2-C6 alkynyl, phenyl, naphthyl, pyridyl, pyrrolyl, thienyl and indolyl; optionally, the phenyl, pyridyl and thienyl are each independently substituted by 1 to 2 (preferably 1) groups selected from the group consisting of R^{x}, or the phenyl is substituted by 2 adjacent groups, and the 2 adjacent groups and the carbon atoms to which they are respectively connected form 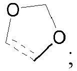
more preferably, R³ is selected from the group consisting of:
1) 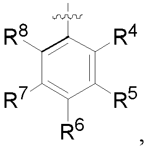 wherein:
R⁴, R⁵, R⁶, R⁷ and R⁸ are each independently selected from the group consisting of hydrogen, C1-C6 alkyl, halogen, C1-C6 alkoxy, C1-C6 haloalkyl, C2-C6 alkynyl, cyano, phenyl and -NR^{m}Rⁿ; or, R⁴, R⁵ and the carbon atoms to which they are respectively connected together form a 5- to 6-membered heterocyclic ring, and R⁶, R⁷ and R⁸ are hydrogen;
preferably, any one of R⁴, R⁵, R⁶, R⁷ and R⁸ (e.g., R⁵, R⁶ or R⁷) is selected from the group consisting of hydrogen, C1-C6 alkyl, halogen, C1-C6 alkoxy, C1-C6 haloalkyl, C2-C6 alkynyl, cyano, phenyl and -NR^{m}Rⁿ, and the rest are hydrogen;
more preferably, any one of R⁴, R⁵, R⁶, R⁷ and R⁸ (e.g., R⁵, R⁶ or R⁷) is selected from the group consisting of hydrogen, methyl, fluorine, chlorine, iodine, methoxy, 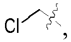 trifluoromethyl, 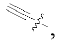 cyano, phenyl and 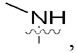 and the rest are hydrogen;
or preferably, R⁴, R⁵ and the carbon atoms to which they are respectively connected together form 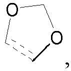 and R⁶, R⁷ and R⁸ are hydrogen;
2) 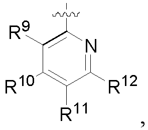 wherein:
R⁹, R¹⁰, R¹¹ and R¹² are each independently selected from the group consisting of hydrogen, C1-C6 alkyl, halogen and C1-C6 alkoxy;
preferably, R⁹, R¹⁰, R¹¹ and R¹² are each independently selected from the group consisting of hydrogen, halogen and C1-C6 alkoxy;
more preferably, any one of R⁹, R¹⁰, R¹¹ and R¹² (e.g., R¹⁰ or R¹¹) is selected from the group consisting of hydrogen, halogen and C1-C6 alkoxy, and the rest are hydrogen;
most preferably, any one of R⁹, R¹⁰, R¹¹ and R¹² (e.g., R¹⁰ or R¹¹) is selected from the group consisting of hydrogen, chlorine and methoxy, and the rest are hydrogen;
3) 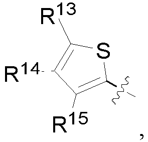 wherein:
R¹³, R¹⁴ and R¹⁵ are each independently selected from the group consisting of hydrogen, C1-C6 alkyl and halogen;
preferably, any one of R¹³, R¹⁴ and R¹⁵ (e.g., R¹³) is selected from the group consisting of hydrogen, C1-C6 alkyl and halogen, and the rest are hydrogen;
more preferably, any one of R¹³, R¹⁴ and R¹⁵ (e.g., R¹³) is selected from the group consisting of hydrogen, methyl and chlorine, and the rest are hydrogen;
4) 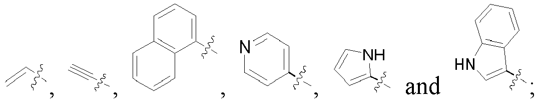
most preferably, R³ is selected from the group consisting of 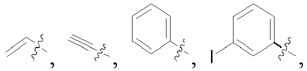 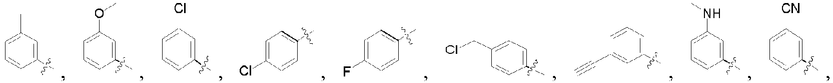 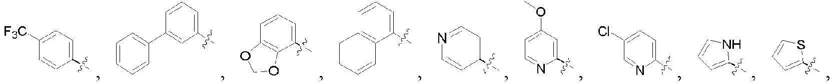 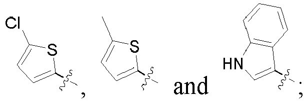
preferably, R^{x} is selected from the group consisting of methyl, fluorine, chlorine, iodine, methoxy, 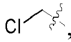 trifluoromethyl, 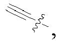 cyano, phenyl and -NR^{m}Rⁿ;
preferably, among R^{m} and Rⁿ, any one is hydrogen, and the other is selected from the group consisting of C1-C6 alkyl;
preferably, among R^{m} and Rⁿ, any one is hydrogen, and the other is methyl.

5. The compound, or stereoisomer, prodrug, crystal form, pharmaceutically acceptable salt, pharmaceutically acceptable solvate or pharmaceutically acceptable ester of the compound, according to any one of claims 1 to 4, wherein R^{4'} is selected from the group consisting of phenyl-(CH₂)ᵣ- and quinolyl-(CH₂)ᵣ-, and optionally, the phenyl and quinolyl are each independently substituted by a group selected from the group consisting of R^{a};
preferably, R^{4'} is selected from the group consisting of phenyl-(CH₂)ᵣ- and quinolyl-(CH₂)ᵣ-, and optionally, the phenyl is substituted by a group selected from the group consisting of R^{a};
preferably, r is selected from the group consisting of 0 and 1;
preferably, R^{a} is selected from the group consisting of halogen, C1-C6 alkyl-O-C(=O)-, and piperazinyl which is substituted by 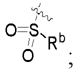
more preferably, R^{a} is selected from the group consisting of halogen, C1-C6 alkyl-O-C(=O)-, and piperazinyl which is substituted by 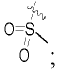
most preferably, R^{a} is selected from the group consisting of bromine, 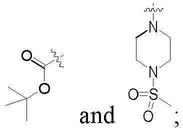 preferably, R^{b} is selected from the group consisting of C1-C6 alkyl;
more preferably, R^{b} is methyl;
more preferably, R^{4'} is selected from the group consisting of 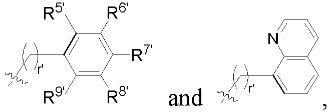 wherein,
r' is selected from the group consisting of 0, 1, 2, 3, 4 and 5;
preferably, r' is selected from the group consisting of 0 and 1;
R^{5'}, R^{6'}, R^{7'}, R^{8'} and R^{9'} are each independently selected from the group consisting of hydrogen, halogen, C1-C6 alkyl-O-C(=O)-, and 5- to 6-membered saturated heterocyclyl optionally substituted by 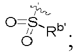
preferably, among R^{5'}, R^{6'}, R^{7'}, R^{8'} and R^{9'}, any one (e.g., R^{7'}) is selected from the group consisting of hydrogen, halogen, C1-C6 alkyl-O-C(=O)-, and 5- to 6-membered saturated heterocyclyl optionally substituted by 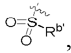 and the rest are hydrogen;
more preferably, among R^{5'}, R^{6'}, R^{7'}, R^{8'} and R^{9'} , any one (e.g., R^{7'}) is selected from the group consisting of hydrogen, halogen, C1-C6 alkyl-O-C(=O)-, and piperazinyl which is substituted by 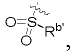 and the rest are hydrogen;
further preferably, among R^{5'}, R^{6'}, R^{7'}, R^{8'} and R^{9'}, any one (e.g., R^{7'}) is selected from the group consisting of hydrogen, halogen, C1-C6 alkyl-O-C(=O)-, and piperazinyl which is substituted by 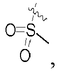 and the rest are hydrogen;
most preferably, among R^{5'}, R^{6'}, R^{7'}, R^{8'} and R^{9'}, any one (e.g., R^{7'}) is selected from the group consisting of hydrogen, bromine, 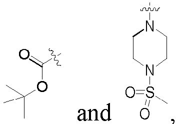 and the rest are hydrogen;
R^{b'} is selected from the group consisting of hydrogen and C1-C6 alkyl;
preferably, R^{b'} is selected from the group consisting of C1-C6 alkyl;
more preferably, R^{b'} is methyl;
most preferably, R^{4'} is selected from the group consisting of

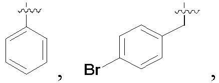

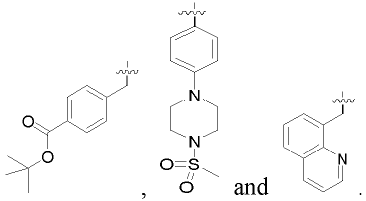

6. The compound, or stereoisomer, prodrug, crystal form, pharmaceutically acceptable salt, pharmaceutically acceptable solvate or pharmaceutically acceptable ester of the compound, according to any one of claims 1 to 5, wherein L¹ is selected from the group consisting of 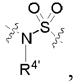 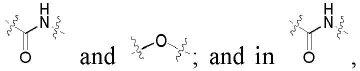 the carbonyl end is connected to L², and the amino end is connected to the benzene ring; and in 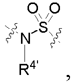 the amino end is connected to L², and the acyl end is connected to the benzene ring;
preferably, L¹ is 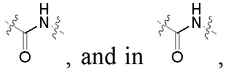 the carbonyl end is connected to L², and the amino end is connected to the benzene ring.

7. The compound, or stereoisomer, prodrug, crystal form, pharmaceutically acceptable salt, pharmaceutically acceptable solvate or pharmaceutically acceptable ester of said compound, according to any one of claims 1 to 6, wherein L² is selected from the group consisting of 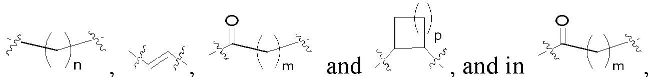 the carbonyl end is connected to R³;
preferably, L² is selected from the group consisting of 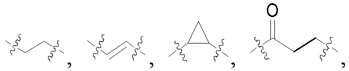 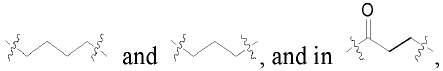
the carbonyl end is connected to R³;
more preferably, L² is selected from the group consisting of 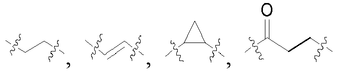 and 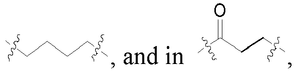 the carbonyl end is connected to R³;
most preferably, L² is 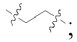
preferably, n is selected from the group consisting of 2, 3 and 4;
preferably, n is selected from the group consisting of 2 and 4;
preferably, m is selected from the group consisting of 1, 2 and 3;
preferably, m is 2;
preferably, p is 0.

8. The compound, or stereoisomer, prodrug, crystal form, pharmaceutically acceptable salt, pharmaceutically acceptable solvate or pharmaceutically acceptable ester of the compound, according to any one of claims 1 to 7, wherein the structural formula of the compound represented by Formula I is selected from the group consisting of the following: 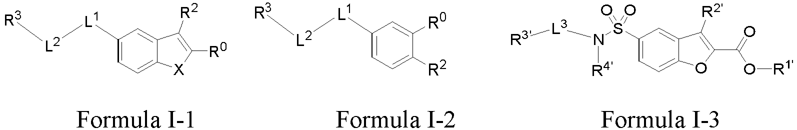 wherein:
X is selected from the group consisting of O, S and NH, preferably O;
R⁰, R², R³, L¹ and L² are defined in any one according to claims 1 to 7;
R^{1'} is selected from the group consisting of hydrogen and C1-C6 alkyl;
preferably, R^{1'} is selected from the group consisting of hydrogen and ethyl;
more preferably, R^{1'} is selected from the group consisting of hydrogen;
R^{2'} is selected from the group consisting of hydrogen and C1-C6 alkyl;
preferably, R^{2'} is selected from the group consisting of C1-C6 alkyl;
more preferably, R^{2'} is methyl;
R^{3'} is phenyl, optionally, the phenyl is substituted by 1 to 5 groups selected from the group consisting of C1-C6 alkyl;
preferably, R^{3'} is phenyl, optionally, the phenyl is substituted by 1 group selected from the group consisting of C1-C6 alkyl;
more preferably, R^{3'} is phenyl;
L³ is selected from 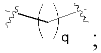
q is selected from the group consisting of 1, 2, 3, 4, 5 and 6;
preferably, q is selected from the group consisting of 1, 2 and 3;
more preferably, q is 2;
R^{4'} is selected from the group consisting of phenyl-(CH₂)ᵣ- and 5- to 10-membered heteroaryl-(CH₂)ᵣ-, optionally, the phenyl and 5- to 10-membered heteroaryl are each independently substituted by 1 to 5 groups selected from the group consisting of R^{a};
preferably, R^{4'} is selected from the group consisting of phenyl-(CH₂)ᵣ- and quinolyl-(CH₂)ᵣ-, optionally, the phenyl and quinolyl are each independently substituted by 1 group selected from the group consisting of R^{a};
more preferably, R^{4'} is selected from the group consisting of phenyl-(CH₂)ᵣ- and quinolyl-(CH₂)ᵣ-, optionally, the phenyl is substituted by 1 group selected from the group consisting of R^{a};
r is selected from the group consisting of 0, 1, 2, 3, 4 and 5;
preferably, r is selected from the group consisting of 0 and 1;
R^{a} is selected from the group consisting of halogen, C1-C6 alkyl-O-C(=O)-, and 5- to 6-membered saturated heterocyclyl optionally substituted by 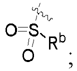
preferably, R^{a} is selected from the group consisting of halogen, C1-C6 alkyl-O-C(=O)-, and piperazinyl which is substituted by 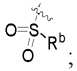
more preferably, R^{a} is selected from the group consisting of halogen, C1-C6 alkyl-O-C(=O)-, piperazinyl which is substituted by 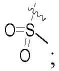
most preferably, R^{a} is selected from the group consisting of 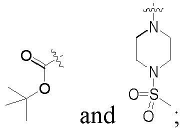
R^{b} is selected from the group consisting of hydrogen and C1-C6 alkyl;
preferably, R^{b} is selected from the group consisting of C1-C6 alkyl;
more preferably, R^{b} is methyl;
further preferably, R^{4'} is selected from the group consisting of 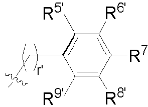 and 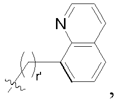 wherein,
r' is selected from the group consisting of 0, 1, 2, 3, 4 and 5;
preferably, r' is selected from the group consisting of 0 and 1;
R⁵', R^{6'}, R^{7'}, R^{8'} and R^{9'} are each independently selected from the group consisting of hydrogen, halogen, C1-C6 alkyl-O-C(=O)-, and 5- to 6-membered saturated heterocyclyl optionally substituted by 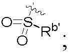
preferably, among R^{5'}, R^{6'}, R^{7'}, R^{8'} and R^{9'}, any one (e.g., R^{7'}) is selected from the group consisting of hydrogen, halogen, C1-C6 alkyl-O-C(=O)-, and 5- to 6-membered saturated heterocyclyl optionally substituted by 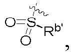 and the rest are hydrogen;
more preferably, among R^{5'}, R^{6'}, R^{7'}, R^{8'} and R^{9'} , any one (e.g., R^{7'}) is selected from the group consisting of hydrogen, halogen, C1-C6 alkyl-O-C(=O)-, piperazinyl which is substituted by 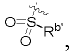 and the rest are hydrogen;
further preferably, among R^{5'}, R^{6'}, R^{7'}, R^{8'} and R^{9'}, any one (e.g., R^{7'}) is selected from the group consisting of hydrogen, halogen, C1-C6 alkyl-O-C(=O)-, and piperazinyl which is substituted by 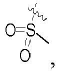 and the rest are hydrogen;
most preferably, among R^{5'}, R^{6'}, R^{7'}, R^{8'} and R^{9'}, any one (e.g., R^{7'}) is selected from the group consisting of hydrogen, bromine, 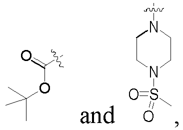 and the rest are hydrogen;
R^{b'} is selected from the group consisting of hydrogen and C1-C6 alkyl;
preferably, R^{b'} is selected from the group consisting of C1-C6 alkyl;
more preferably, R^{b'} is methyl;
most preferably, R^{4'} is selected from the group consisting of

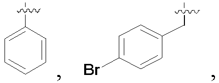

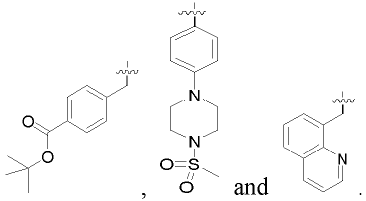

9. The compound, or stereoisomer, prodrug, crystal form, pharmaceutically acceptable salt, pharmaceutically acceptable solvate or pharmaceutically acceptable ester of the compound, according to claim 8, wherein the structural formula of the compound represented by Formula I-1 is: 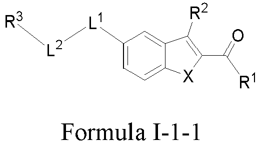 wherein:
X is selected from the group consisting of O, S and NH, preferably O;
R¹, R², R³, L¹ and L² are defined in any one according to claims 1 to 7.

10. The compound, or stereoisomer, prodrug, crystal form, pharmaceutically acceptable salt, pharmaceutically acceptable solvate or pharmaceutically acceptable ester of the compound, according to claim 8, wherein the structural formula of the compound represented by Formula I-2 is: 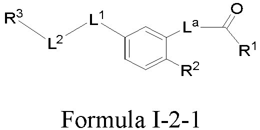 wherein:
R¹, R², R³, L^{a}, L¹ and L² are defined in any one according to claims 1 to 7;
preferably, R¹ is hydroxyl;
preferably, R² is selected from the group consisting of halogen;
more preferably, R² is fluorine;
preferably, R³ is 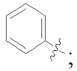
preferably, L^{a} is selected from the group consisting of C1-C6 alkylene;
more preferably, L^{a} is selected from the group consisting of 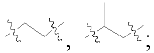
preferably, L¹ is selected from the group consisting of 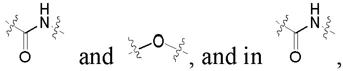 the carbonyl end is connected to L², and the amino end is connected to the benzene ring;
more preferably, L¹ is 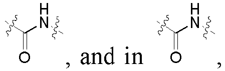 the carbonyl end is connected to L², and the amino end is connected to the benzene ring;
preferably, L² is selected from 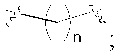
more preferably, L² is selected from the group consisting of 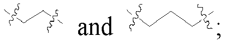
preferably, n is selected from the group consisting of 1, 2, 3, 4, 5 and 6;
more preferably, n is selected from the group consisting of 2 and 3.

11. The compound, or stereoisomer, prodrug, crystal form, pharmaceutically acceptable salt, pharmaceutically acceptable solvate or pharmaceutically acceptable ester of the compound, according to any one of claims 1 to 10, wherein the compound is selected from the group consisting of the following:
| No. | Structural Formula | No. | Structural Formula |
|---|---|---|---|
| 1 | 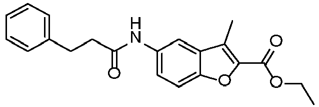 | 16 | 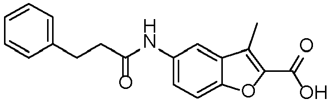 |
| 2 | 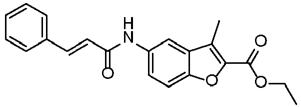 | 17 | 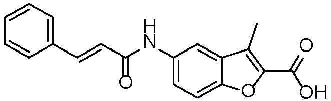 |
| 3 | 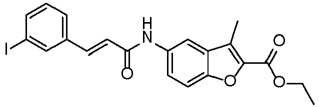 | 18 | 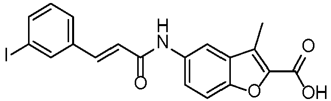 |
| | | | |
|---|---|---|---|
| 4 | 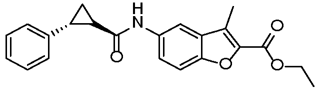 | 19 | 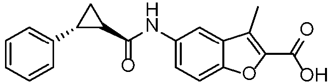 |
| 5 | 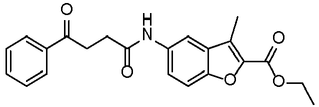 | 20 | 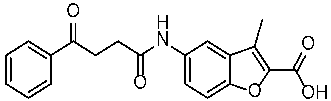 |
| 6 | 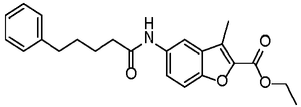 | 21 | 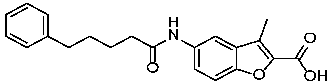 |
| 7 | 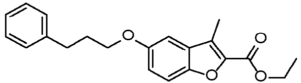 | 22 | 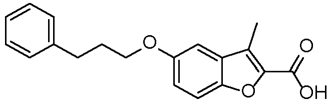 |
| B101 | 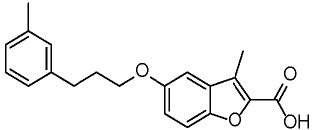 | B113 | 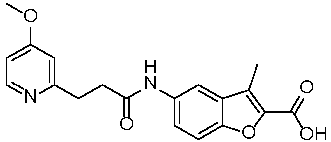 |
| B102 | 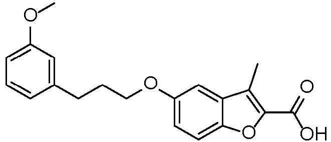 | B114 | 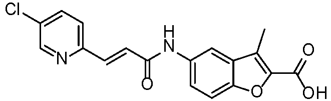 |
| B103 | 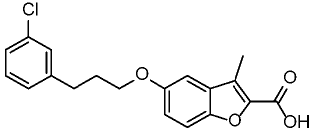 | B115 | 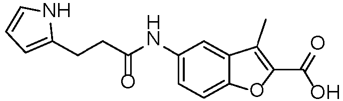 |
| GPR132-A-8 | 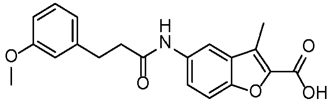 | B116 | 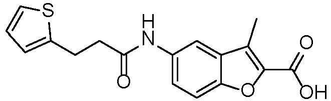 |
| B104 | 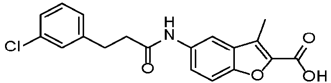 | B117 | 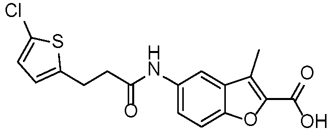 |
| B105 | 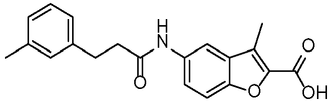 | B118 | 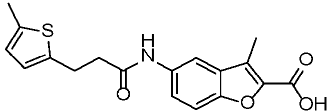 |
| | | | |
|---|---|---|---|
| B106 | 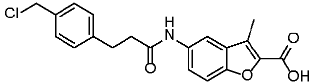 | B119 | 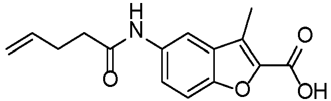 |
| B107 | 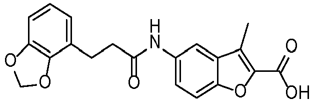 | B120 | 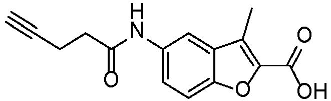 |
| B108 | 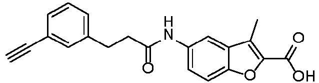 | B121 | 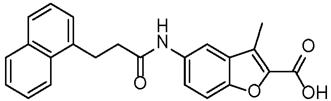 |
| B109 | 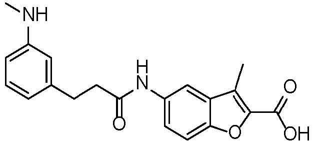 | B122 | 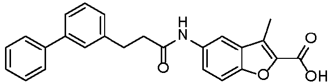 |
| B110 | 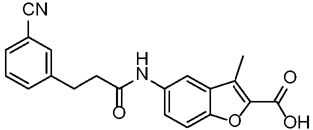 | B123 | 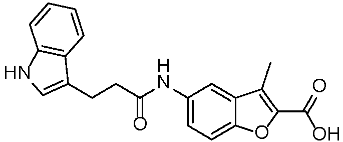 |
| B111 | 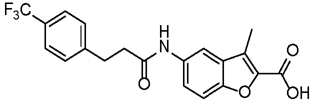 | B124 | 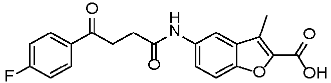 |
| B112 | 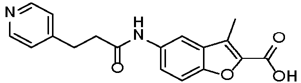 | B125 | 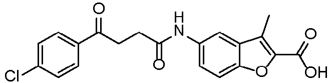 |
| B126 | 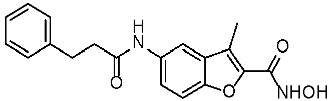 | B127 | 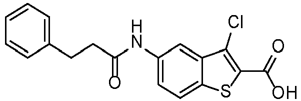 |
| B128 | 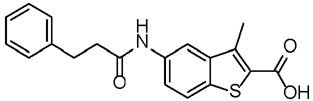 | B129 | 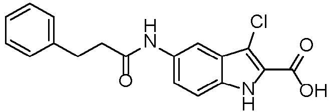 |
| B130 | 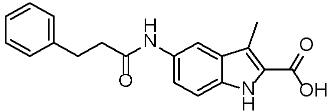 | B131 | 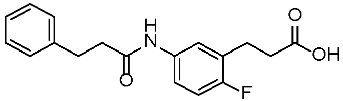 |
| | | | |
|---|---|---|---|
| B132 | 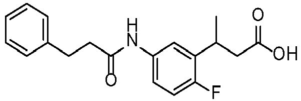 | B133 | 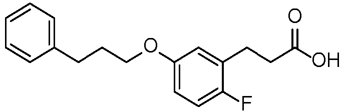 |
| 9 | 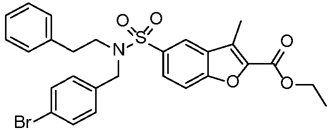 | 24 | 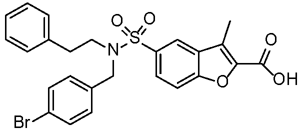 |
| 10 | 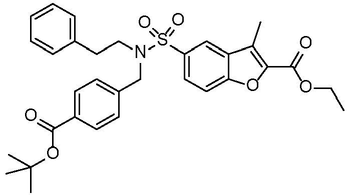 | 25 | 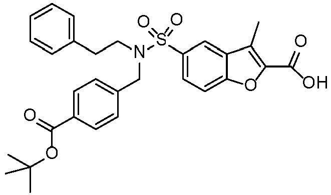 |
| 11 | 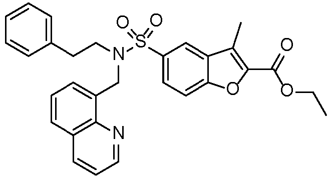 | 26 | 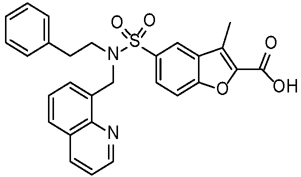 |
| 12 | 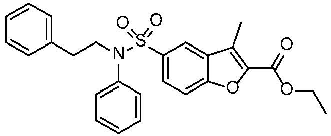 | 27 | 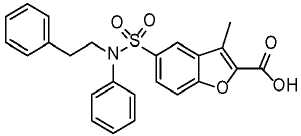 |
| 14 | 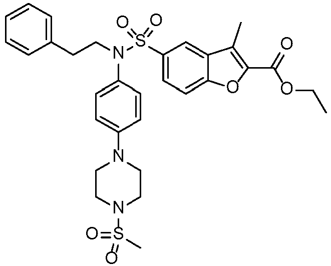 | 29 | 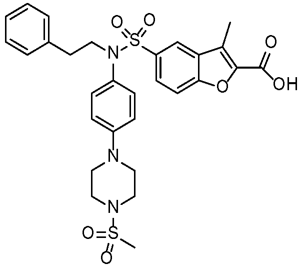 |

12. A pharmaceutical composition, which comprises the compound, or stereoisomer, prodrug, crystal form, pharmaceutically acceptable salt, pharmaceutically acceptable solvate or pharmaceutically acceptable ester of the compound, according to any one of claims 1 to 11, and an optional pharmaceutically acceptable excipient.

13. Use of the compound, or stereoisomer, prodrug, crystal form, pharmaceutically acceptable salt, pharmaceutically acceptable solvate or pharmaceutically acceptable ester of the compound, according to any one of claims 1 to 11, in manufacture of a medicament; or use of the pharmaceutical composition according to any one of claim 12 in manufacture of a medicament; or use of the compound shown in the following table, or stereoisomer, prodrug, crystal form, pharmaceutically acceptable salt, pharmaceutically acceptable solvate or pharmaceutically acceptable ester of the compound, in the manufacture of a medicament, wherein the medicament is used for treatment and/or prevention of a disease related to GPR132, or for hematopoietic stem cell transplantation, or for bone marrow transplantation;
| No. | Structural Formula | No. | Structural Formula |
|---|---|---|---|
| 8 | 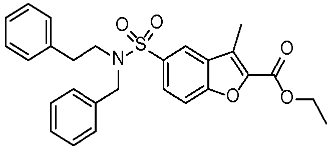 | 23 | 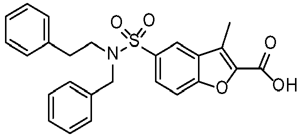 |
| 13 | 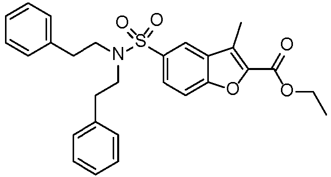 | 28 | 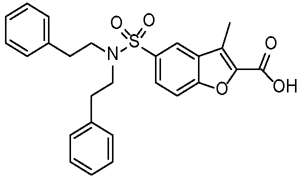 |
| 15 | 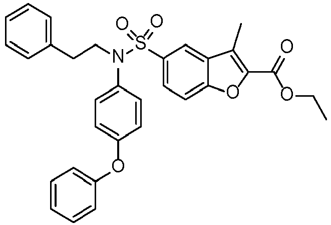 | 30 | 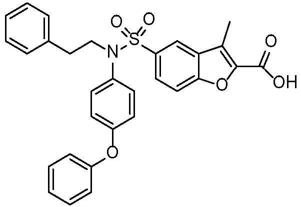 |
preferably, the disease related to GPR132 is selected from the group consisting of tumor, metabolic disease, immune-related disease, and neuropathic pain;
preferably, the tumor is selected from the group consisting of breast cancer, melanoma, meningioma, soft tissue sarcoma, salivary gland tumor, liver cancer (e.g., primary liver cancer), intraspinal tumor, mediastinal tumor, brain cancer, bone cancer, penile cancer, osteosarcoma, intracranial tumor, tongue cancer, maxillary sinus cancer, thyroid cancer, malignant lymphoma, multiple myeloma, pituitary adenoma, testicular tumor, non-Hodgkin's lymphoma, bladder cancer, leukemia (e.g., acute myeloid leukemia), gastric cancer, nasopharyngeal cancer, laryngeal cancer, oral cancer, esophageal cancer, lung cancer, kidney cancer, cervical cancer, choriocarcinoma, vulvar cancer, skin cancer, endometrial cancer, ovarian cancer, prostate cancer, pancreatic cancer, colon cancer, rectal cancer, colorectal cancer, Kaposi's sarcoma, non-melanoma skin cancer (including squamous cell carcinoma and basal cell carcinoma), hemangioma, and glioma;
preferably, the metabolic disease is selected from the group consisting of atherosclerosis, obesity, non-alcoholic fatty liver disease (NAFLD) (e.g., simple fatty liver or non-alcoholic steatohepatitis (NASH)), metabolic syndrome, type 2 diabetes, type 1 diabetes, insulin resistance, hyperinsulinemia, glucose intolerance, hyperglycemia, hyperlipidemia (e.g., hypercholesterolemia), and secondary complications of these diseases (e.g., complications of diabetes, such as retinopathy, neuropathy, nephropathy and delayed wound healing, or cardiovascular and cerebrovascular diseases such as atherosclerosis, coronary heart disease, hypertension, and stroke);
preferably, the immune-related disease is selected from the group consisting of:
secondary immunodeficiency: for example, secondary immunodeficiency caused by infection (e.g., rubella, measles, leprosy, tuberculosis, cytomegalovirus infection, HIV infection, coccidioidomycosis infection), protein loss (e.g., nephrotic syndrome, protein-losing enteropathy), insufficient immunoglobulin synthesis, lymphocyte loss (e.g., lymphocyte loss caused by drug and/or systemic infection), secondary immunodeficiency caused by other diseases (e.g., diabetes, cirrhosis, subacute sclerosing panencephalitis) and/or immunosuppressive therapy, and
autoimmune disease: for example, inflammatory bowel disease, systemic lupus erythematosus, rheumatoid arthritis, scleroderma, hyperthyroidism, juvenile diabetes, idiopathic thrombocytic purpura, autoimmune hemolytic anemia, colorectal inflammation (e.g., ulcerative colitis), skin disease, chronic liver disease.
